# DEMANDE DE BREVET EUROPEEN

(11) **EP 2 226 314 A1**
(43) Date de publication de la demande: **08.09.2010**
(21) Numéro de dépôt: 09305201.7
(22) Date de dépôt: 04.03.2009
(51) Int. Cl.: C07D 207/46

(54) **Agents de reticulation**

(71) Demandeur: CENTRE NATIONAL DE LA RECHERCHE SCIENTIFIQUE (CNRS), 75016 Paris (FR)
(72) Inventeur: Martinez, Jean, 34720, CAUX (FR); Subra, Gilles, 34980, SAINT GELY DU FESC (FR); Goubet, Christine, 34270, LES MATELLES (FR); Paramelle, David, 81160, SAINT JUERY (FR); Forest, Eric, 38120, SAINT-EGREVE (FR); Heymann, Michaël, 68110, ILLZACH (FR); Geourjon, Christophe, 69007, LYON (FR)
(74) Mandataire: Ahner, Francis

(57) **Abrégé**

L'invention concerne Agent de réticulation des protéines de formule (I) 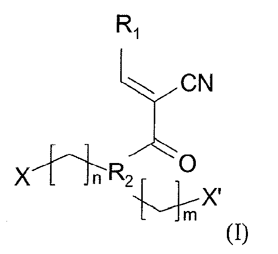 dans laquelle R₁ est un groupe aryle éventuellement substitué une ou plusieurs fois par un groupement choisi dans le groupe constitué des groupements hydroxy, C₁-C₄ alkyle, OBoc, SO₃Na, Deu, C₁-C₄ alcoxy,
R₂ est N, 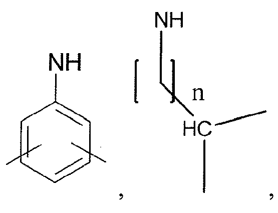 ou 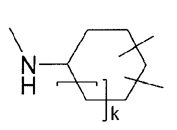 n et m sont des entiers identiques ou différents compris antre 0 et 10, p est un entier compris entre 0 et5, k est 0, 1, 2 ou 3, X et X', identiques ou différents, sont une fonction réactive des protéines ; ainsi qu'une méthode d'analyse structurale d'une protéine ou d'un complexe de protéines.

## Description

L'invention concerne le domaine des analyses structurales des protéines par spectrométrie de masse.

L'analyse des structures tridimensionnelles des protéines et de leurs interactions avec leur environnement (*e.g.* ADN, protéines, membranes) est très importante pour la compréhension des fonctions biologiques des protéines dans leur milieu naturel.

Afin d'obtenir ces données structurales à haute résolution, la RMN et la DRX sont des méthodes d'analyse couramment utilisées. Cependant, ces méthodes sont très limitées par certaines contraintes : cristallisation de protéine, utilisation de grande quantité de matériel (5 à 10 mg de protéines).

L'utilisation d'agents de réticulation chimique conjointement à la spectrométrie de masse est une méthode de plus en plus fréquente pour la détermination de données structurales à basse résolution permettant de s'affranchir des contraintes précédentes.

Cette approche consiste à effectuer une digestion enzymatique des protéines du milieu réactionnel après avoir réalisé la réaction de réticulation en solution. Le mélange de peptides obtenu est alors analysé par spectrométrie de masse afin d'identifier les peptides modifiés par les agents de réticulation. Des données structurales sont alors obtenues de cette identification. Cette stratégie a été utilisée dans le cadre d'études structurales à basse résolution de protéines ou d'interactions protéine-protéine.

Les agents de réticulation chimique possèdent une ou deux fonctions réactives reliées par un bras espaceur. Ces fonctions réactives sont capables de réagir avec les chaînes latérales des aminoacides de protéines. Après identification des résidus aminoacides des protéines modifiés par les deux fonctions réactives, des contraintes de distances au sein de ces protéines peuvent être déterminées grâce à la longueur connue du bras espaceur de l'agent de réticulation chimique.

Des agents homobifonctionnels, tels que les esters bis-imido ou les halogénures dialkyles furent mis au point et utilisés sur des protéines dès les années 1950.

Toutefois, l'utilisation d'agents de réticulation des protéines en solution pose un problème majeur de détection de peptides d'intérêts dans un milieu mixte complexe comprenant à la fois les peptides modifiés et non modifiés par les agents de réticulation.

En effet, au nombre important de type de produits de réticulation, s'ajoute la complexité du mélange peptidique résultant de la digestion enzymatique. Par conséquent, l'intensité des signaux des peptides d'intérêt est souvent très faible. Pour palier à cette difficulté, de nombreuses stratégies mettant en jeu le marquage d'agents de réticulation chimique ont été développées. Elles permettent soit l'enrichissement des peptides réticulés à l'aide de méthodes de purification soit la discrimination de leurs signaux lors des analyses par spectrométrie de masse. Parmi ces méthodes, on peut citer :

### - Marquage isotopique

L'expérience de réticulation est effectuée avec un mélange équimolaire de deux agents de réticulation de structure identique mais dont l'un des deux est modifié par des isotopes stables. Les isotopes utilisés sont le deutérium et l'oxygène 18. Ce type de marquage aura pour effet de créer un dédoublement du signal du peptide réticulé grâce à la différence de masse entre les deux types d'agents de réticulation ce qui permet de faciliter la détection de peptides modifiés.

### - Agent de réticulation clivable

L'utilisation d'une fonction clivable au sein de l'agent de réticulation permet de faciliter l'identification des peptides réticulés par comparaison entre le spectre de masse de tous les signaux de peptides d'intérêt et celui obtenu après clivage de l'agent de réticulation. Il est ainsi possible d'identifier les signaux ayant été atteints par le clivage et donc d'en déduire les masses des produits modifiés par l'agent de réticulation.

Le clivage peut être induit soit par un traitement chimique ou directement lors d'analyses en spectrométrique de masse en tandem (MS/MS).

### - Marquage fluorescent

L'utilisation d'un agent de réticulation marqué avec un motif fluorescent a pour but de faciliter la détection de peptides réticulés lors de la purification par chromatographie.

### - Purification par affinité

Le marquage d'un agent de réticulation trifonctionnel avec un motif biotine permet d'effectuer une purification par affinité avec des billes d'avidine. Cette méthode permet donc d'enrichir l'échantillon en peptides réticulés après l'étape de digestion enzymatique.

### - Marqueur UV-absorbant

L'utilisation d'un marquage de peptides avec un composé UV-absorbant afin d'améliorer la détection en spectrométrie de masse MALDI-TOF a été divulguée dans la demande WO03/087839. Cette étude a montré que le marquage de peptides avec le marqueur HCCA permet d'augmenter l'intensité de leurs signaux.

Il existe donc un besoin en une nouvelle méthode permettant d'améliorer la sensibilité de détection de peptides d'intérêt en spectrométrie de masse MALDI-TOF et la discrimination de leur signal dans le cas de mélanges complexes, en particulier pour l'étude de structures tridimensionnelles basse résolution de protéines par exemple en identifiant des contraintes de distances entre différentes chaînes latérales.

Le Demandeur a précédemment montré (Lascoux et al., Discrimination and Selective Enhancement of Signals in the MALDI Mass Spectrum of a Protein by Combining a Matrix-Based Label for Lysine Residues with a Neutral Matrix, Angew. Chem. 2007, 119, 5690-5693) que l'utilisation conjointe de l'acide α-cyano-4-hydroxycinnamique (HCCA) comme marqueur de peptides et une matrice neutre de structure similaire, l'α-cyano-4-hydroxycinnamate de méthyle (HCCE), lors des analyses par spectrométrie de masse MALDI-TOF, permet d'augmenter le signal de peptides marqués en faible proportion dans un mélange de peptides non marqués, et d'obtenir un effet de discrimination de leur signal appelé « effet de discrimination HCCA/HCCE ».

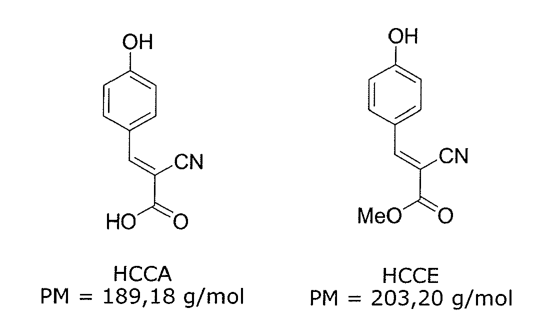

De manière inattendue et surprenante, le Demandeur a maintenant montré que des agents de réticulation bifonctionnels marqués HCCA ou un dérivé de HCCA permettent d'identifier par spectrométrie de masse MALDI-TOF des peptides d'intérêt d'une protéine dans un mélange peptidique complexe par marquage en solution alors que lesdits peptides ne sont pas visibles sur le spectre de masse MALDI-TOF de la protéine. Cet effet sélectif est encore accentué par l'utilisation de la matrice HCCE.

### Résumé de l'invention

L'invention a donc pour objet un agent de réticulation des protéines de formule (I) 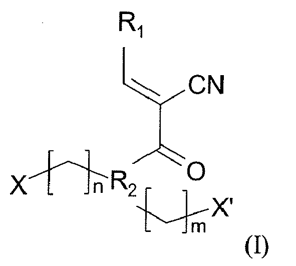 dans laquelle
- R₁: est un groupe aryle éventuellement substitué une ou plusieurs fois par un groupement choisi dans le groupe constitué des groupements hydroxy, C₁-C₄ alkyle, OBoc, SO₃Na, Deu, C₁-C₄ alcoxy,
- R₂ est N,: 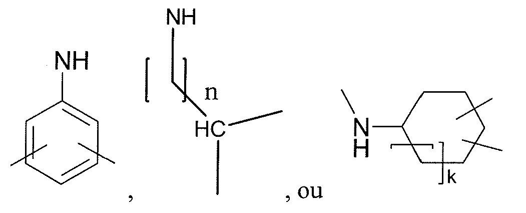
- n et m: sont des entiers identiques ou différents compris entre 0 et 10, de préférence compris entre 1 et 5
p est un entier compris entre 0 et 5,
k est 0, 1, 2 ou 3,
X et X', identiques ou différents, sont une fonction réactive des protéines.

Un autre objet de l'invention est un procédé de préparation d'un agent de réticulation selon l'invention.

Un autre objet de l'invention est l'utilisation d'un agent de réticulation selon l'invention pour l'analyse de la structure tridimensionnelle d'une protéine en spectrométrie de masse.

Un autre objet de l'invention est une méthode d'analyse structurale d'une protéine ou d'un complexe de protéines comprenant les étapes suivantes :
a) réticulation de la protéine ou du complexe protéique sur l'agent de réticulation selon l'une quelconque des revendications 1 à 8 par les fonctions X et/ou X',
b) digestion enzymatique de la protéine ou complexe de protéines fixé à l'agent de réticulation sur selon l'une quelconque des revendications 1 à 8,
c) analyse par spectrométrie de masse.

### Description détaillée

### DEFINITIONS

Par groupement « C₁₋₁₀ alkyle », on entend une chaîne hydrocarbonée saturée, linéaire ou ramifiée, comportant de 1 à 10 atomes de carbone, comme par exemple un groupement, méthyle, éthyle, isopropyle, *tertio*-butyle, pentyle, etc.

Par groupement « C₂₋₁₀ alcényle », on entend une chaîne hydrocarbonée, linéaire ou ramifiée, comportant au moins une insaturation et comportant de 2 à 10 atomes de carbone, comme par exemple un groupement éthényle, propényle, 2,4-hexadiényle, etc.

Par groupement « aryle », on entend un groupement aromatique, comportant de préférence de 5 à 10 atomes de carbone, comprenant un ou plusieurs cycles et comprenant éventuellement un hétéroatome, en particulier un oxygène, un azote ou un soufre, comme par exemple un groupement phényle, furane, indole, pyridine, naphtalène, etc.

Le terme « halogène » désigne un fluor, un brome, un chlore ou un iode.

Boc désigne un groupement protecteur d'amine de formule t-butyloxycarbonyle.

Deu désigne deutérium.

Par groupement « (C₁-C₆)alcoxy », on entend, au sens de la présente invention, un groupe (C₁-C₆)alkyle, tel que défini ci-dessus, lié à la molécule par l'intermédiaire d'un atome d'oxygène. A titre d'exemple, on peut citer les groupes méthoxy, éthoxy, propoxy, isopropoxy, butoxy ou encore tert-butoxy.
tBu désigne tert-butyle.

### LES AGENTS DE RETICULATION

L'agent de réticulation des protéines de l'invention présente la formule (I) 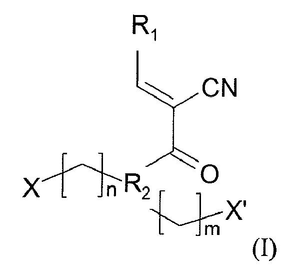 dans laquelle
- R₁: est un groupe aryle éventuellement substitué une ou plusieurs fois par un groupement choisi dans le groupe constitué des groupements hydroxy, C₁-C₄ alkyle, OBoc, SO₃Na, Deu, C₁-C₄ alcoxy,

- R₂: est N,

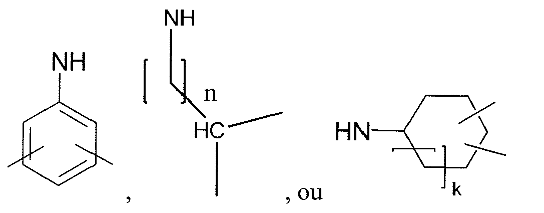

- n et m: sont des entiers identiques ou différents compris antre 0 et 10, de préférence compris entre 1 et 5
p est un entier compris entre 0 et5,
k est 0, 1, 2 ou 3,
X et X', identiques ou différents, sont une fonction réactive des protéines.

Avantageusement, n = m.

De préférence, R₁ est un groupe phényle éventuellement substitué une ou plusieurs fois par un groupement choisi dans le groupe constitué des groupements hydroxy, C₁-C₄ alkyle, OBoc, SO₃Na, Deu, C₁-C₄ alkcoxy.

De manière particulièrement préférée, R₁ est un groupe phénoxy, tout particulièrement un groupe para-hydroxy-phényle.

De préférence, R₂ est N,

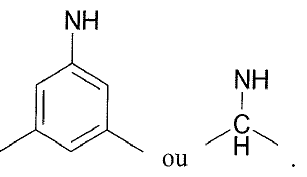

Avantageusement, l'agent de réticulation selon l'invention présente la formule (II), (III) ou (IV)

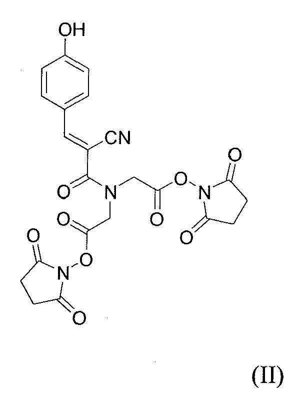

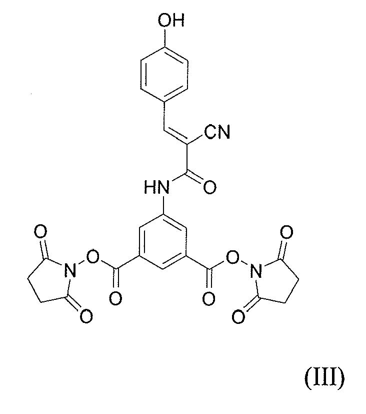

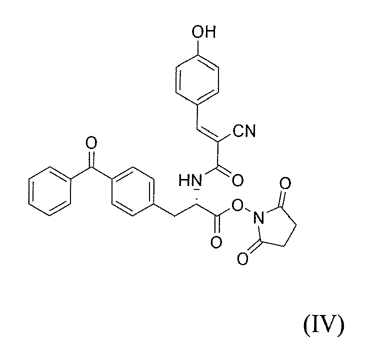

Avantageusement, X et X', identiques ou différents, sont choisis dans le groupe constitué des fonctions imidoester, ester *N*-hydroxysuccinimide, isocyanate, isothiocyanate, *N*-maléimide, disulfure, 1,2-dicarbonyle, benzophénone et arylazide.

L'agent de réticulation de l'invention comprend deux fonctions réactives des protéines X et X'. Il est possible de concevoir une variété très importante d'agents de réticulation suivant la nature des fonctions réactives utilisées qui peuvent être identiques ou pas (*i.e.* agent homo ou hétérobifonctionnel).

On appelle « réticulation de la protéine ou du complexe protéique sur l'agent de réticulation sur support solide de l'invention » la réaction d'une ou plusieurs fonctions réactives X et/ou X' de l'agent de réticulation A avec un ou plusieurs groupements de la protéine ou du complexe protéique résultant en la fixation covalente de ladite protéine ou dudit complexe protéique à l'agent de réticulation A.

Les agents de réticulation homobifonctionnels possèdent deux fonctions réactives identiques pouvant réagir avec le même type de fonction.

Lors d'expériences de réticulation sur des protéines, une des deux fonctions réactives réagit sur une chaîne latérale d'un résidu aminoacide. La seconde fonction réactive réagit ensuite soit de façon intramoléculaire sur une autre chaîne latérale avoisinante, soit sur une chaîne latérale d'un résidu aminoacide appartenant à une autre protéine. Etant donné que les deux fonctions réactives sont identiques, le protocole de réaction se fait en une seule étape.

Les agents de réticulation hétérobifonctionnels possèdent deux fonctions réactives ciblant différents aminoacides.

Ils sont généralement utilisés pour réticuler des protéines en deux étapes (*e.g.* agents de réticulation possédant une fonction réactive non-spécifique et spécifique). Une fois la première fixation effectuée, il est donc possible d'effectuer une purification des protéines modifiées avant d'effectuer la réaction de la deuxième fonction réactive. Ceci peut favoriser les réactions intramoléculaires et peut permettre d'obtenir des données plus diversifiées qu'avec des agents de réticulation homobifonctionnels.

### LES FONCTIONS REACTIVES

X et X' sont des fonctions réactives spécifiques des protéines capables de réagir avec un groupement réactif des protéines.

Avantageusement, X et X', identiques ou différents, sont choisies dans le groupe constitué des fonctions réactives spécifiques des amines, des fonctions réactives spécifiques des acides carboxyliques, des fonctions réactives spécifiques des thiols, des fonctions réactives spécifiques des guanidines, des fonctions réactives non spécifiques.

**Les fonctions réactives spécifiques des amines** réagissent avec les amines primaires présentes sur les protéines. Il existe deux types d'amines primaires au sein d'une protéine : N-terminale et N-ε des lysines. Des études ont montré qu'il est possible de les cibler de façon indépendante grâce à leur différence de valeur de pKa respectif (pKa_{N-terminale} = 8 et pKa_{N-ε des} lysines = 10,5).

Trois types de fonctions sont couramment utilisées dans ce but : les imidoesters, les esters *N-*hydroxysuccinimide (NHS), les isocyanates (et isothiocyanates). Ce sont tous des agents activés électrophiles possédant un bon groupe partant par substitution nucléophile.

**Les fonctions réactives spécifiques des acides carboxyliques** sont présentes dans les résidus Asp, Glu et en partie C-terminale des protéines. Ces fonctions ne sont pas réactives en soi et nécessitent une activation.

Cette activation est généralement effectuée avec des carbodiimides. La *O*-acylurée formée par cette activation permet de réagir avec une amine primaire et de former une liaison amide.

Les fonctions réactives spécifiques des thiols sont les fonctions *N*-maléimide et disulfure.

La fonction thiol est la plus réactive des fonctions nucléophiles au sein d'une protéine.

Cependant, les chaînes latérales des résidus cystéines sont souvent engagées dans des ponts disulfures ce qui empêche leur réaction avec des agents de réticulation chimique. Par conséquent, une réaction de réduction (avec de l'éthanedithiol ou EDT) de ces liaisons est généralement nécessaire afin de retrouver les fonctions thiols libres. Le pKa des thiols des résidus cystéines étant d'environ 8,6, leur réactivité augmente lorsque l'on forme l'ion thiolate à pH supérieur à 8,6.

**Les fonctions réactives spécifiques des guanidines** sont les composés 1,2-dicarbonyl qui réagissent spécifiquement avec les chaînes latérales des arginines.

**Les fonctions réactives non spécifiques** réagissent sur des molécules par exposition à la lumière UV. Un agent idéal photoréactif doit avoir différentes qualités :
- Il a une forte réactivité
- Il est capable de réagir indifféremment sur n'importe quel type de résidu
- Il est stable dans le noir
- Il réagit avec une lumière dont la longueur d'onde ne cause aucun dommage photolytique à l'échantillon biologique
- Le produit de sa réaction doit être stable.

Les mécanismes de réaction de ces fonctions sont généralement radicalaires ce qui permet d'agir indifféremment sur divers résidus. D'une manière générale, les agents de réticulation chimique ayant une fonction réactive non-spécifique possèdent aussi une fonction réactive spécifique. De cette manière, il est possible de cibler des résidus d'intérêt avec une première étape de fixation mettant en jeu la fonction réactive spécifique puis de marquer tous les résidus présents dans un certain périmètre (défini par la longueur du bras espaceur liant les deux fonctions réactives) grâce à l'action de la fonction réactive non-spécifique.

Il existe deux types de fonctions couramment utilisées dans ce but : les benzophénones et les arylazides.

### PROCEDE DE PREPARATION

Les agents de réticulation de l'invention sont préférentiellement préparés de la façon suivante.

Le procédé de préparation des agents de réticulation de l'invention comprend trois étapes.

La première étape de ces trois étapes consiste en l'étape (a) ou (a').

L'étape (a) consiste en un couplage peptidique entre l'amine RR'NH et l'acide carboxylique 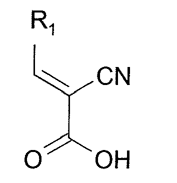 dont la fonction acide est éventuellement activée
pour donner 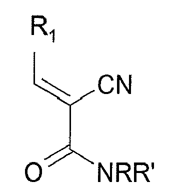 avec R¹ ayant la signification donnée précédemment
avec R et R' représentant :
- tous deux -(alkyl)-COOY
- l'un des deux H et l'autre 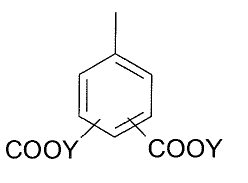
- l'un des deux H et l'autre 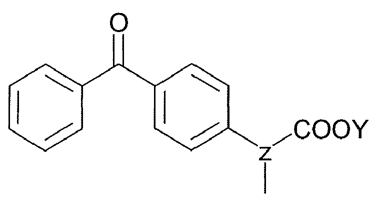
avec Y est H ou tBu.
où Z signifie un groupe alkyle en C₁ à C₈

L'étape (a') comprend les étapes (i), (ii) et (iii)
L'étape (i) consiste en la réaction de RR'NH avec 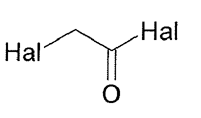 où Hal est un atome d'halogène
   en présence d'une base pour donner 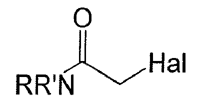
L'étape (ii) consiste en la réaction de 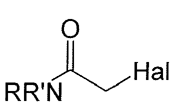 avec un cyanure, tel que KCN, pour donner 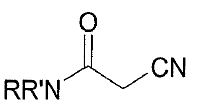
L'étape (iii) consiste en la réaction de 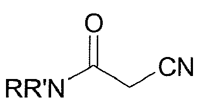 avec R₁CHO en présence d'une base pour donner

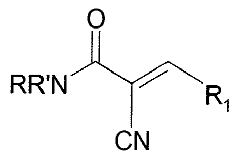

L'étape (b) consiste en l'hydrolyse éventuelle de l'ester obtenu à l'étape (a) ou (a') en acide pour donner 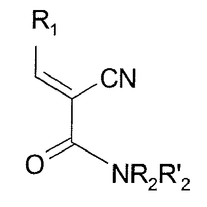 avec R₂ et R₂' représentant :
- tous deux -(alkyl)-COOH
- l'un des deux H et l'autre 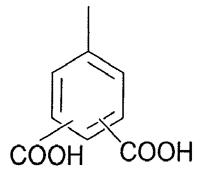
- l'un des deux H et l'autre 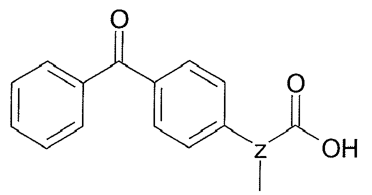
où Z signifie un groupe alkyle en C₁ à C₈

L'étape (c) consiste en la réaction du composé obtenu à l'étape (b) précédente pour obtenir une fonction réactive des protéines.

Cette fonction réactive des protéines est préférentiellement une fonction ester NHS.

Un couplage peptidique avec un acide carboxylique est réalisé de préférence en présence d'un agent de couplage, tel que le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-H-benzotriazole-1-yl)-1,1,3,3-tétraméthyluronium hexafluorophosphate (HBTU), le 2-(1H-benzotriazole-1-yl)-1,1,3,3-tétraméthyluronium tétrafluoroborate (TBTU) ou encore le O-(7-azobenzotriazol-1-yl)-1,1,3,3-tétraméthyluronium hexafluorophosphate (HATU), éventuellement associé à un auxiliaire de couplage tel que le N-hydroxy succinimide (NHS), le *N*-hydroxy benzotriazole (HOBt), le 3,4-dihydro-3-hydroxy-4-oxo-1,2,3-benzotriazole (HOOBt), le 1-hydroxy-7-azabenzotriazole (HOAt) ou le *N-*hydroxysylfosuccinimide (sulfo NHS).

Les réactions de protection/déprotection des fonctions amine et les réactions de couplage peptidique sont des réactions bien connues de l'homme du métier.

### METHODE DE RETICULATION DE PROTEINES EN SOLUTION POUR ANALYSE STRUCTURALE PAR SPECTROMETRIE DE MASSE

### ❖ Réticulation de protéine

L'agent de réticulation chimique en solution est mis en présence de la protéine dissoute.

Avantageusement, l'agent de réticulation chimique supporté est mis en présence de la protéine dissoute dans un tampon salin afin de s'approcher des conditions physiologiques.

Il en résulte une fixation covalente de la protéine sur l'agent de réticulation par l'intermédiaire des fonctions réactives X et/ou X'

### ❖ Digestion enzymatique

Les protéines réticulées sont digérées. Pour ce faire, le mélange est mis en présence d'une enzyme (*e.g.* la trypsine) solubilisée, de préférence dans un tampon salin.

### ❖ Analyse par spectrométrie de masse

### SPECTROMETRIE DE MASSE

De préférence, l'analyse par spectrométrie de masse est effectuée par spectrométrie MALDI-TOF.

Les échantillons sont déposés soit en goutte séchée, soit sur couche mince.

Le dépôt en goutte séchée est préféré. Il est réalisé par co-cristallisation d'une goutte de solution matricielle et d'une goutte d'une solution d'analyte.

### LOGICIEL D'ANALYSE DES DONNEES DE SPECTROMETRIE DE MASSE

Avantageusement, les données de l'analyse par spectrométrie de masse des mélanges de peptides sont analysées par un logiciel.

En effet, l'analyse par spectrométrie de masse des mélanges de peptides fournis par l'étape de digestion enzymatique apporte généralement une quantité importante de données. Afin de faciliter l'identification des peptides détectés lors de ces expériences, de nombreux logiciels de prédiction ont été créés (*e.g.* Pro-CrossLink, VirtualMSLab, SearchXLink et MS2Assign).

Le but de ces logiciels est de fournir rapidement une liste de peptides potentiels non modifiés ou modifiés par l'agent de réticulation utilisé, correspondant à chaque valeur m/z du spectre de masse. Les valeurs expérimentales sont comparées aux valeurs théoriques calculées à partir d'une digestion *in silico* du modèle protéique étudié.

Dans le cadre de la présente invention, un logiciel de prédiction, appelé MSX-3D, a été créé au sein de l'Institut de Biologie et de Chimie des Protéines (IBCP) à Lyon.

### MATRICES MALDI-TOF

La matrice HCCA est couramment utilisée lors d'analyses par spectrométrie de masse MALDI-TOF de peptides. Elle est capable d'absorber la lumière UV du laser de la source MALDI. L'énergie qu'elle absorbe est restituée sous forme d'énergie thermique et permet la désorption des composés co-cristallisés avec la matrice.

L'ester α-cyano-4-hydroxycinnamate de méthyle, appelé HCCE ou α-CNME, est une matrice relativement peu utilisée en analyse par spectrométrie de masse MALDI-TOF. C'est l'ester méthylique de la matrice HCCA. Du fait de leurs structures proches, elles présentent toutes deux des caractéristiques similaires :
- Poids moléculaires (189 g/mol pour l'HCCA et 203 g/mol pour l'HCCE)
- Mêmes aspects physiques : poudres jaunes cristallines
- Structures aromatiques similaires leur conférant des propriétés d'absorption comparables.

Cependant, il existe un facteur important pouvant les différencier : leur capacité à donner un proton. En effet, la matrice HCCA possède deux fonctions acides de pKa distincts : la fonction acide carboxylique de pka₁ égal à 2 et la fonction alcool phénolique de pKa₂ égal à 8. Par contre, la matrice HCCE ne possède qu'une seule fonction acide : la fonction alcool phénolique de pKa₁ égal à 8.

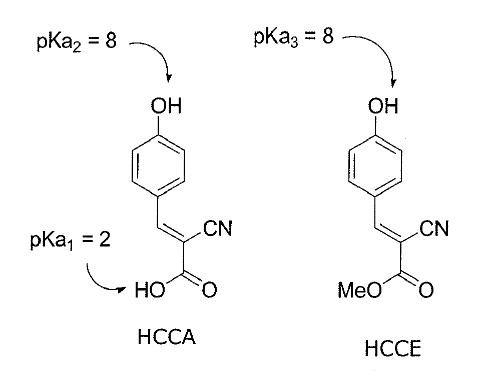

La matrice HCCA possède donc une fonction acide carboxylique permettant de jouer un rôle de donneur de proton lors du processus d'ionisation impliqué dans la source MALDI : c'est pourquoi elle est appelée matrice acide. La matrice HCCE est beaucoup moins efficace dans ce même rôle car elle ne possède qu'une seule fonction faiblement acide (*i.e.* alcool phénolique de pKa₃ = 8). Elle est donc appelée matrice neutre.

### FIGURES

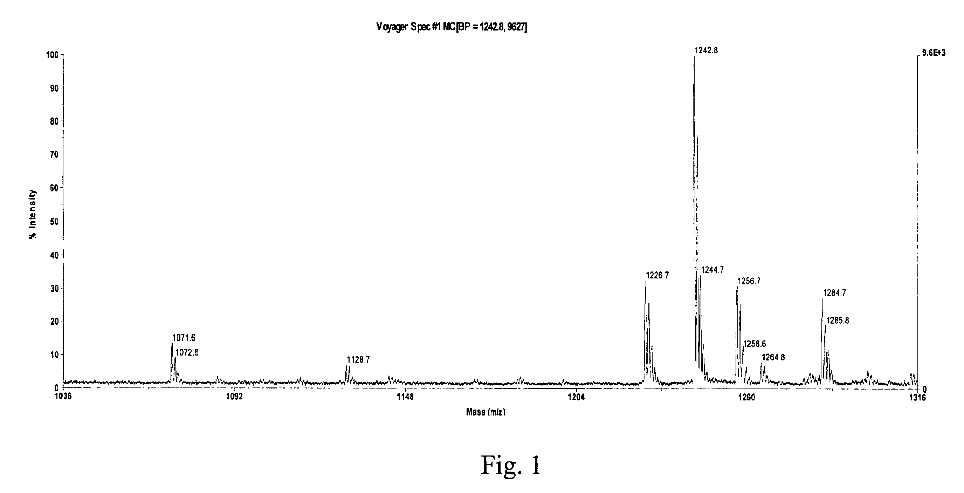

Figure 1 : Analyse d'un mélange équimolaire des cinq peptides modèles JMV3346 à JMV3350 avec la matrice HCCA.
Figure 2 : Spectre de masse du mélange peptidique issu de la digestion trypsique du cytochrome c non modifié (avec matrice HCCA).
Figure 3 : Spectre de masse de la digestion trypsique après réticulation du cytochrome c à 100 µM avec R = 0,5 (avec matrice HCCA) : 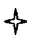 signal de peptide potentiellement modifié par le JMV3378.
Figure 4 : Spectre de masse de la digestion trypsique après réticulation du cytochrome c à 100 µM avec R = 0,5 (avec matrice neutre HCCE) : 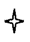 signal de peptide potentiellement modifié par le JMV3378.
Figure 5 : Spectre de masse du mélange peptidique issu de la digestion trypsique de l'apomyoglobine non modifiée (avec matrice HCCA).
Figure 6 : Spectre de masse de la digestion trypsique après réticulation de l'apomyoglobine à 100 µM avec R = 0,2 (avec matrice HCCA) : 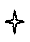 signal de peptide potentiellement modifié par l'agent de réticulation JMV3378.
Figure 7 : Spectre de masse de la digestion trypsique après réticulation de l'apomyoglobine à 100 µM avec R = 0,2 (avec matrice neutre HCCE) : 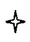 signal de peptide potentiellement modifié par l'agent de réticulation JMV3378.

Les exemples suivants illustrent l'invention sans en limiter la portée.

### EXEMPLES

### Exemple 1 : Influence de la fonction alcool du marqueur HCCA

Les cinq peptides modèles sont des décapeptides amidés en position C-terminale possédant la même séquence aminoacide dans le but de ne pas influencer leur capacité d'ionisation intrinsèque en spectrométrie de masse MALDI-TOF. Ils ont été synthétisés sur support solide en stratégie Fmoc. Un premier peptide JMV3346 n'est pas marqué et sert de référence pour évaluer l'effet de discrimination spectrale. Les peptides modèles JMV3347, JMV3348 et JMV3349 ont été synthétisés par couplage de l'HCCA et deux dérivés en position 4 (*i.e.* acide α-cyanocinnamique ou CCA, acide α-cyano-4-méthoxycinnamique) avec le décapeptide entièrement protégé et fixé sur le support solide. Le peptide JMV3350 a été obtenu par acétylation de la fonction alcool phénolique de l'HCCA couplé au décapeptide entièrement protégé et fixé sur le support solide.

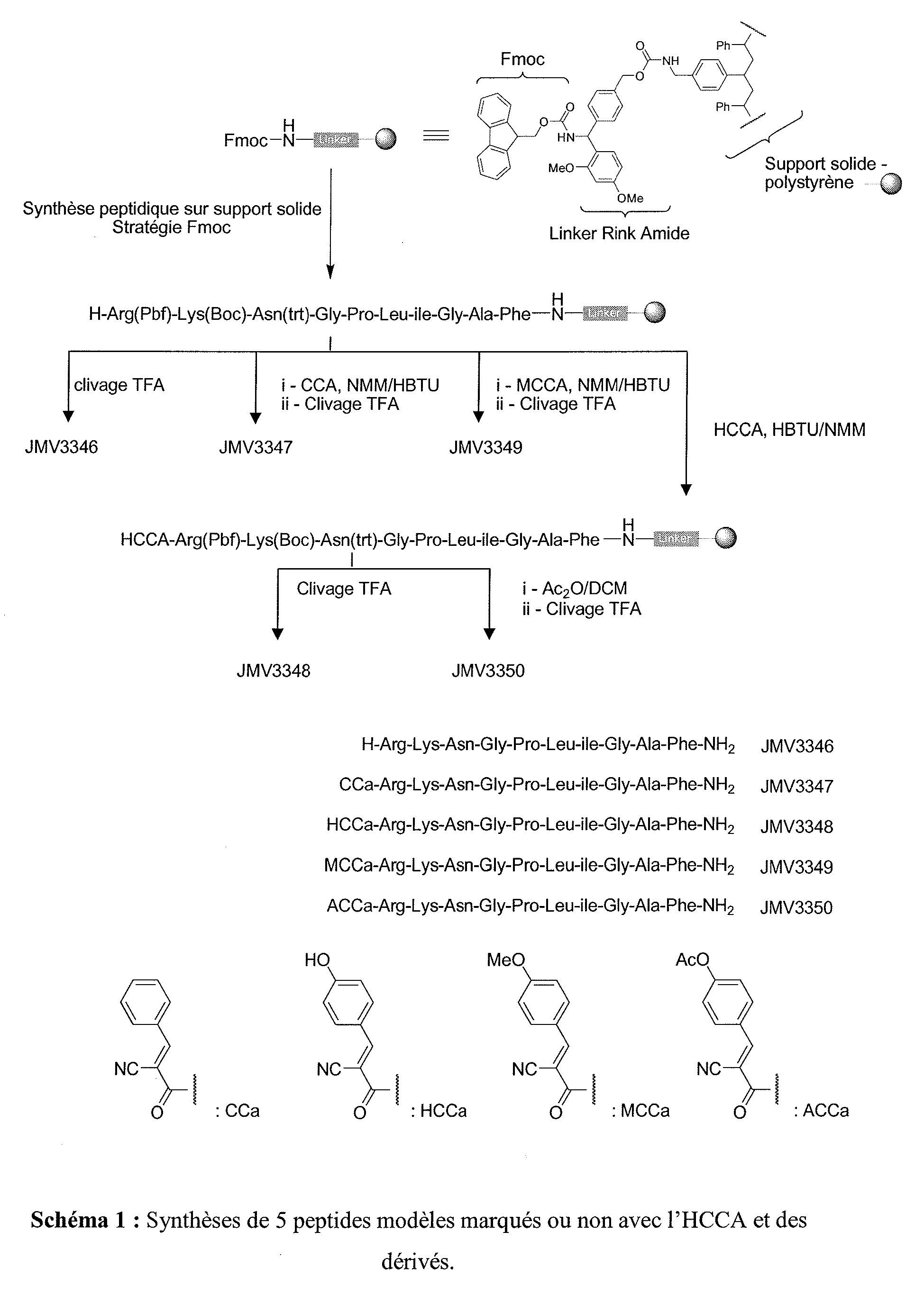

Pour cela, une analyse d'un mélange équimolaire des cinq peptides modèles (JMV3346 à JMV3350) a été effectuée avec la matrice HCCA.

Cette analyse montre que le marqueur HCCA permet d'obtenir une augmentation optimale de l'intensité de signal. De plus, malgré une légère amélioration de la détection des peptides JMV3347, JMV3349 et JMV3350 par rapport au peptide non-marqué, on observe que l'absence de la fonction alcool phénolique ou sa modification par méthylation ou acétylation engendre une forte diminution (environ 2/3 du signal par rapport au signal du JMV3348) de la détection des peptides marqués. Cette diminution peut être due à une hydrophobicité supérieure des marqueurs CCA, MCCA et ACCA induite par la perte du proton de la fonction alcool.

### Exemple 2 : Elaboration d'agents de réticulation bifonctionnels marqués par l'HCCA

### 2-1 - Synthèse d'un agent homobifonctionnel

### 2 - 1 - a - Agent JMV3378 avec un motif iminodiacétique

Le *N*-(α-cyano-4-hydroxycinnamyl)iminodiacétate de *N-*hydroxysuccinimidyle ou JMV3378 a été préparé (Formule (II)).

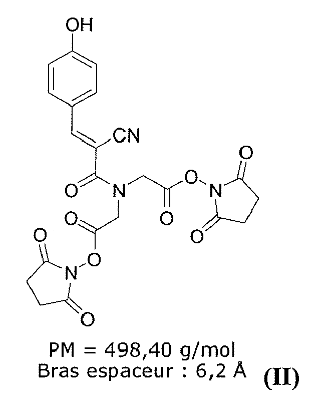

Le couplage direct entre la fonction acide carboxylique de l'HCCA et l'amine secondaire de l'ester iminodiacétate de diéthyle a tout d'abord été envisagé afin de synthétiser le JMV3378.

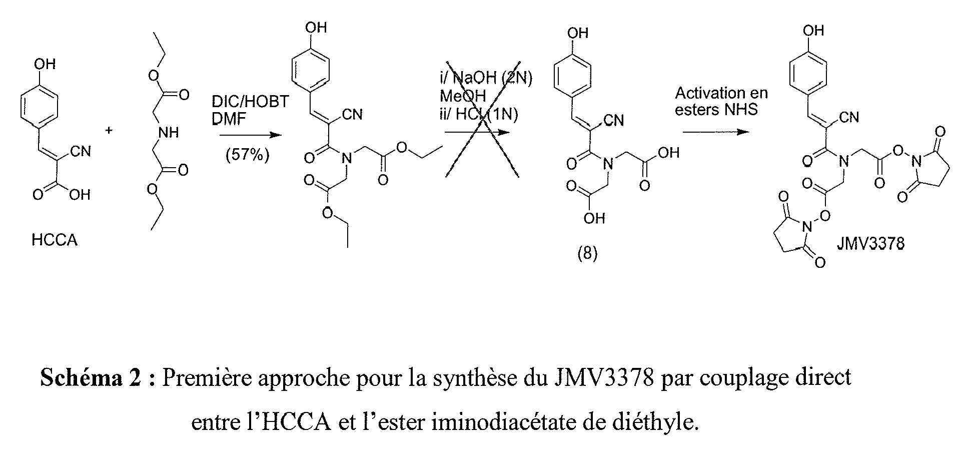

Cette voie de synthèse n'a pu aboutir. En effet, l'étape de saponification a entraîné une dégradation du diester de départ.

Afin d'éviter l'étape de saponification des esters éthyliques, une nouvelle approche utilisant les esters *tert*-butyles a été envisagée (cf. Schéma **3**). Ces esters sont facilement hydrolysables dans une solution d'acide trifluoroacétique (TFA). Contrairement aux traitements basiques, l'HCCA ne semble pas être sensible au TFA. En effet, les préparations d'échantillons avec l'HCCA en spectrométrie de masse MALDI-TOF ainsi que les peptides modèles marqués avec l'HCCA (JMV3348) ont supporté l'utilisation de solutions concentrées de TFA.

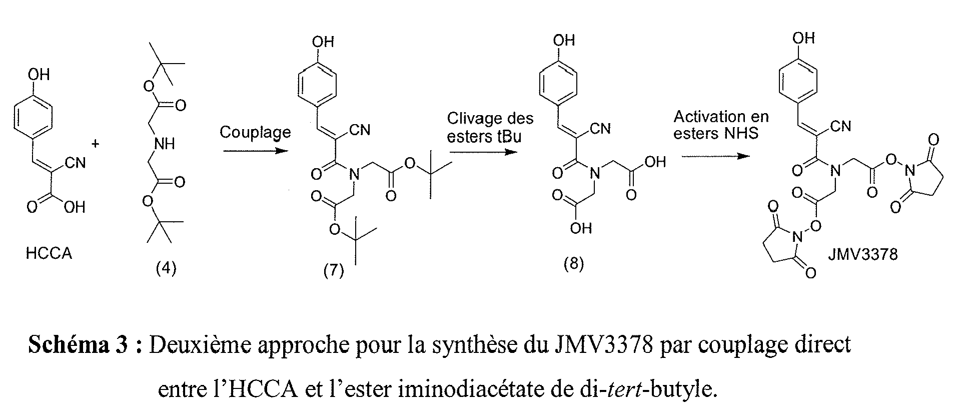

Le composé clef de cette approche est l'ester iminodiacétate de di-*tert*-butyle **4.** Deux voies de synthèse ont été élaborées (cf. Schéma 4).

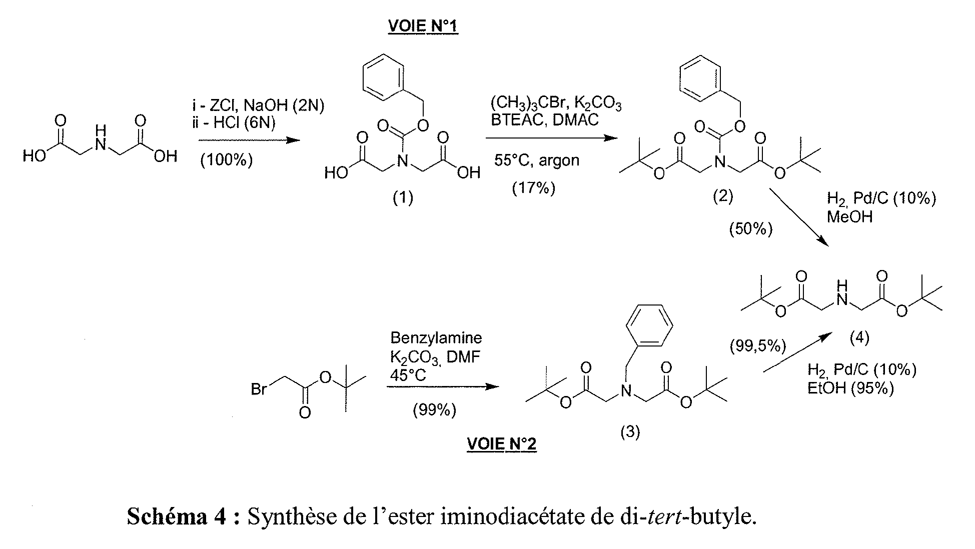

La voie N°1 comporte trois étapes. L'amine secondaire du diacide iminodiacétique est tout d'abord protégée avec un groupement benzyloxycarbonyle afin d'obtenir le composé **1.** Cette réaction est effectuée à l'aide d'une solution de chloroformiate de benzyle dans la soude avec un rendement quantitatif. Une réaction d'estérification du composé **1** effectuée à 55°C dans une solution de diméthylacétamide (DMAC) contenant du chlorure de benzyltriéthylammonium, du bicarbonate de potassium et du bromure de *tert*-butyle, a permis d'obtenir le composé **2** avec un rendement de 17%. Enfin, la déprotection de l'amine permettant d'aboutir au composé **4,** a été réalisée avec un rendement de 50% grâce à une hydrogénolyse effectuée dans du méthanol avec un bullage d'hydrogène catalysé par du palladium sur charbon.

La voie N°2 ne compte que deux étapes. Le composé **3** est obtenu directement par une double substitution nucléophile de l'amine primaire de la benzylamine sur deux molécules de 2-bromoacétate de *tert*-butyle. Cette réaction est réalisée avec un rendement de 99% dans une solution de diméthylformamide (DMF) contenant du bicarbonate de potassium à 45°C. L'hydrogénolyse du groupe benzyle conduisant au composé **4** a été effectuée quantitativement à l'aide d'un bullage d'hydrogène et de palladium sur charbon dans de l'éthanol à 95%.

La voie N°2 semble être plus intéressante car elle permet d'obtenir le diester final **4** en deux étapes de très bon rendement et utilise un ester *tert*-butylique commercial. La préparation de l'ester *tert*-butyle est l'étape limitante dans la voie N°1. D'autre part, cette voie compte une étape en plus et ne permet pas d'obtenir le diester avec un aussi bon rendement que la voie N°2.

L'élaboration de ces deux voies a pu permettre d'envisager une voie de synthèse permettant d'obtenir des agents homologues (cf. Schéma **5**). Dans ce cas, l'étape d'estérification est nécessaire car les esters *tert*-butyliques homologues supérieurs du 2-bromoacétate ne sont pas commerciaux.

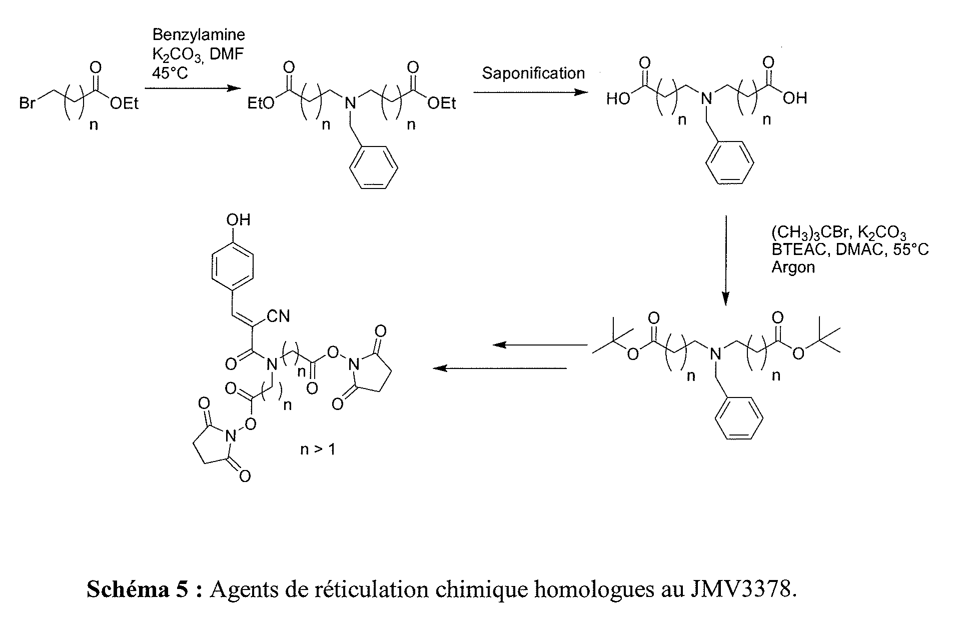

La synthèse du composé **7** a tout d'abord été envisagée par couplage direct entre l'acide HCCA et l'amine secondaire du composé **4** (cf. Schéma **6**).

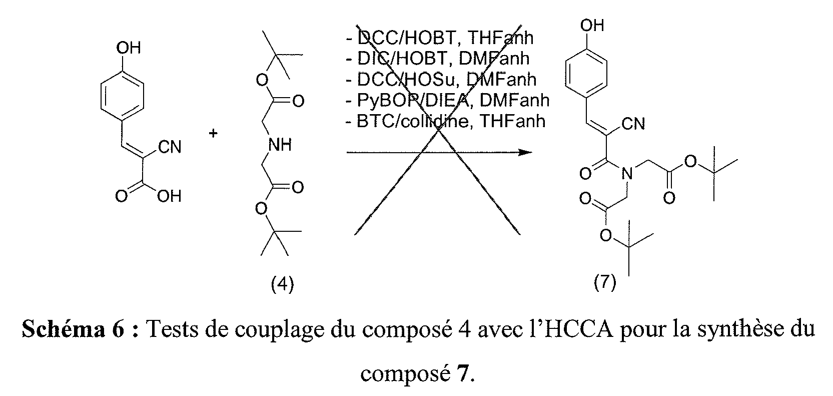

Différentes méthodes de couplage ont été testées mais aucune n'a permis d'aboutir à la formation du composé **7**. Deux hypothèses peuvent être faites pour expliquer cet échec. L'encombrement des deux esters *tert*-butyle peut entraîner une difficulté pour le couplage de l'amine. De plus, l'alcool phénolique de l'HCCA peut potentiellement être engagé dans des réactions secondaires intermoléculaires et réagir avec un ester actif d'une autre molécule.

Une autre approche a donc été élaborée basée sur une condensation de Knoevenagel. Cette fois, le motif HCCA a été synthétisé en trois étapes à partir du composé **4** (cf. Schéma 7).

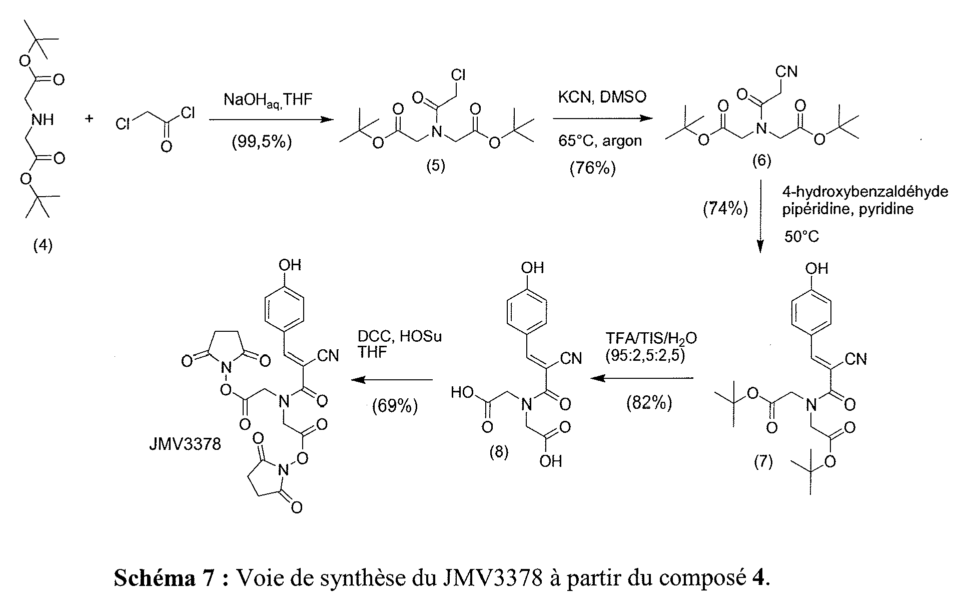

Le composé **4** est d'abord acylé avec du chlorure de 2-chloroacétyle dans un mélange de soude et de tétrahydrofurane (THF) de manière quantitative. La substitution du chlore avec du cyanure de potassium dans du diméthylsulfoxyde (DMSO) à 65°C permet d'obtenir le composé **6.** Une réaction de Knoevenagel entre le composé **6** et du 4-hydroxybenzaldéhyde effectuée dans un mélange de pyridine et de pipéridine à 50°C fournit le composé **7** avec un rendement de 74%. Lors de cette réaction, l'aldéhyde peut être utilisé en large excès afin d'optimiser le rendement de la réaction. L'excès d'aldéhyde à la fin de la réaction peut être retiré facilement en fin de réaction par extraction liquide/liquide dans une phase aqueuse saturée en sulfite de sodium acidifiée par ajout d'acide acétique (pH ~ 5) Les esters *tert*-butyles du composé **7** sont hydrolysés dans un mélange de TFA/TIS/eau (95 : 2,5 : 2,5) afin d'obtenir le diacide **8** avec un rendement de 82%. Le triisopropylsilane (TIS) est un piégeur (ou scavenger) des carbocations *tert*-butyliques. Il permet d'éviter une réaction secondaire d'alkylation de Friedel-Craft en position 3,3' du phénol. Enfin, le JMV3378 est synthétisé grâce à une activation des deux acides carboxyliques avec de la dicyclohexylcarbodiimide (DCC) et de la *N*-hydroxysuccinimide dans du THF avec un rendement de 69%. Les conditions de purification de ce composé sont identiques à celles du JMV3155.

L'agent de réticulation chimique JMV3378 a donc été obtenu grâce à une voie de synthèse en sept étapes avec un rendement global de 31 %.

### 2 - 1 - b - Agent de réticulation chimique avec un motif 5-aminoisophtalique

Un deuxième agent de réticulation homobifonctionnel possédant deux fonctions esters NHS a été synthétisé : le 5-(α-cyano-4-hydroxycinnamido)isophthalate de *N*-hydroxysuccinimidyle (cf. schéma 8). Cet agent de réticulation possède un bras espaceur d'une longueur similaire à celui du composé JMV3378, cependant sa structure est rigide à cause du cycle aromatique isophtalique. Cette rigidité peut permettre d'obtenir de nouvelles informations structurales lors d'expériences de réticulation sur des protéines.

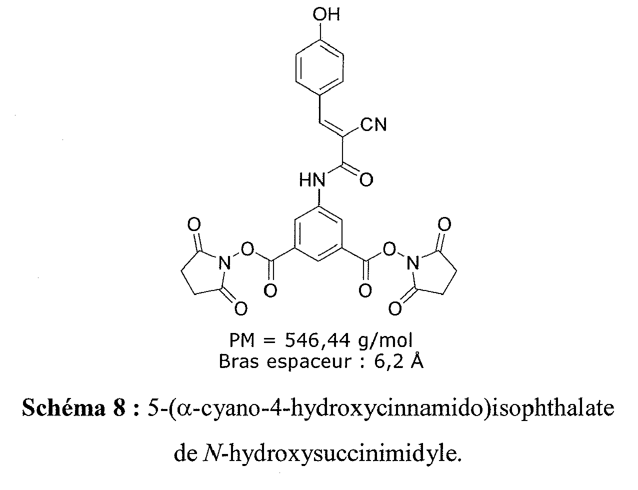

Afin de synthétiser ce composé, une voie similaire à celle développée pour la synthèse du JMV3378 a été utilisée (cf. Schéma **9**).

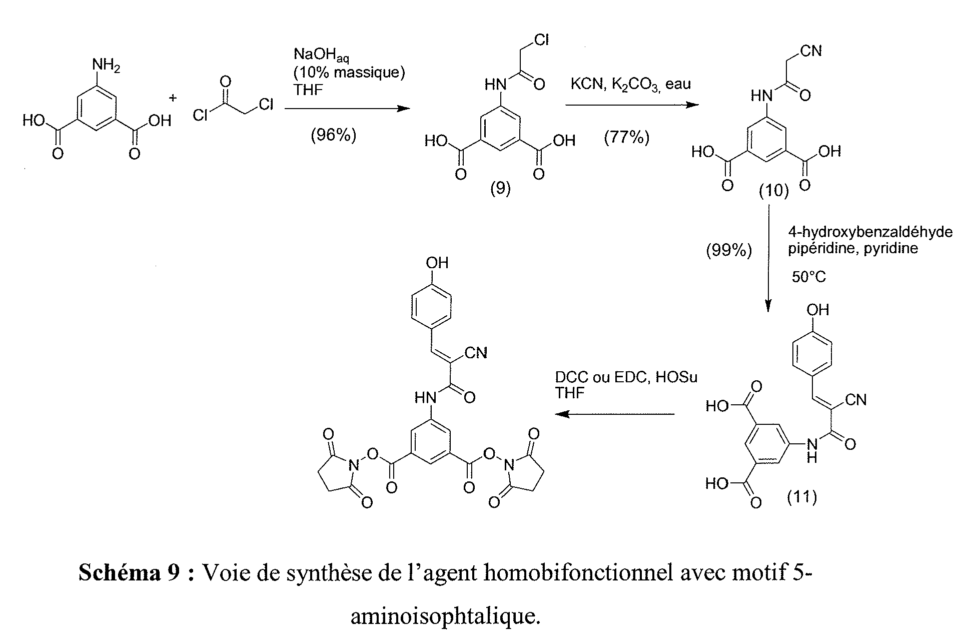

La voie de synthèse est directement initiée par l'acylation du diacide 5-aminoisophtalique avec du chlorure de 2-chloroacétyle dans un mélange de soude et de THF. Le composé **9** est récupéré grâce à une précipitation instantanée dans de l'éther de pétrole avec un rendement de 96%. Une substitution de l'atome de chlore du composé **9** avec du cyanure de potassium dans de l'eau en présence de bicarbonate de potassium permet d'obtenir le composé **10** avec un rendement de 77%. Le diacide **11** est ensuite obtenu avec un rendement de 99% grâce à une réaction de Knoevenagel avec du 4-hydroxybenzaldéhyde dans un mélange de pyridine et de pipéridine à 50°C.

Enfin, le composé est synthétisé grâce à une activation des deux acides carboxyliques avec de la dicyclohexylcarbodiimide (DCC) et de la *N*-hydroxysuccinimide dans du THF. Les conditions de purification de ce composé sont identiques à celles du JMV3155.

### 2-2 - Synthèse d'un agent hétérobifonctionnel

Un agent de réticulation hétérobifonctionnel possédant une fonction réactive non spécifique peut permettre d'obtenir de nombreuses informations plus diversifiées qu'un agent de réticulation homobifonctionnel.

Un agent de réticulation possédant à la fois une fonction ester NHS et une fonction photoactivable benzophénone a été synthétisé : le (S)-3-(4-benzoylphényl)-2-(α-cyano-4-hydroxycinnamido)propanoate de *N*-hydroxysuccinimidyle ou JMV3480 (cf. schéma 10).

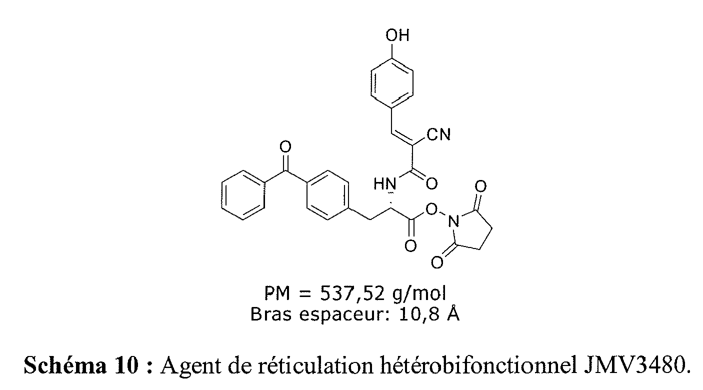

La voie de synthèse de ce composé est présentée dans le schéma 11 ci-dessous.

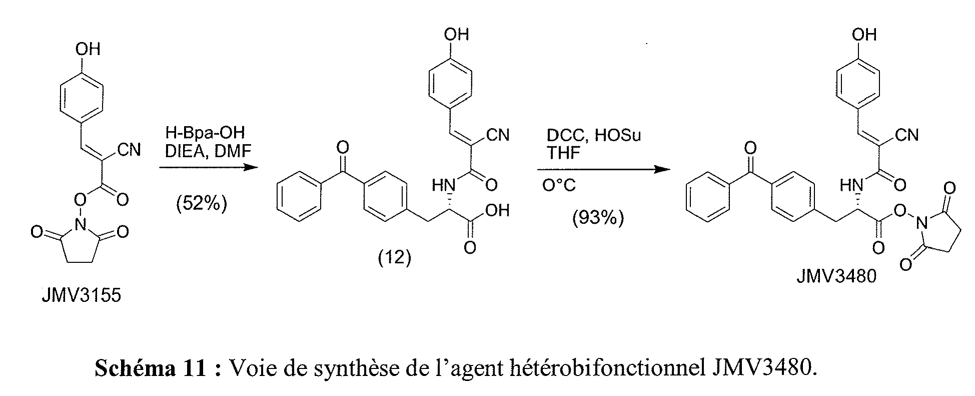

Un couplage aminoacide entre l'agent monofonctionnel JMV3155 et l'acide (S)-*p*-benzoylphénylalanine dans une solution de DMF contenant de la *N,N-*diisopropyléthylamine (DIEA) conduit au composé **12** avec un rendement de 52%. Enfin, l'activation de la fonction acide en ester NHS conduisant au JMV3480, a été effectuée avec de la DCC et du *N*-hydroxysuccinimide dans du THF à 0°C avec un rendement de 93%.

Ces différentes synthèses ont donc permis d'obtenir deux nouveaux agents de réticulation chimique marqués avec le motif UV-absorbant HCCA : un premier agent homobifonctionnel possédant deux fonctions esters NHS spécifiques aux amines primaires - le JMV3378 - et un agent hétérobifonctionnel possédant une fonction ester NHS et une fonction non spécifique benzophénone - le JMV3480.

### Exemple 3 : Applications de l'agent de réticulation JMV3378 à l'étude de protéines modèles

Le cytochrome c et l'apomyoglobine de coeur de cheval ont été utilisées comme protéines modèles.

Plusieurs expériences de réticulation, faisant varier la concentration en protéine et le ratio molaire R = [JMV3378]/[Lys+Nter], ont été réalisées afin d'évaluer les conditions optimales pouvant permettre d'obtenir à la fois un taux de réticulation satisfaisant et un bon effet de discrimination de détection des peptides réticulés par rapport aux peptides non modifiés après digestion enzymatique.

Pour chaque test, la protéine a été dissoute dans un tampon PBS à pH 7,5 et l'agent de réticulation a été dissous dans du DMSO. Le composé JMV3378 a été introduit dans le milieu protéique avec un ratio volumique PBS/DMSO (95 : 5) afin de ne pas perturber la structure de la protéine. Chaque expérience de réticulation a été effectuée à température ambiante pendant 2 minutes avant d'être congelée ou directement analysée.

Après réticulation, les échantillons de protéines ont été analysés par spectrométrie de masse MALDI-TOF en utilisant la matrice acide sinapinique en dépôt de type goutte séchée. Après vérification du taux de réticulation obtenu, les échantillons ont été digérés avec de la trypsine. Chaque mélange peptidique obtenu a été analysé par spectrométrie de masse avec la matrice acide HCCA en dépôt de type goutte séchée et avec la matrice HCCE en dépôt de type couche mince.

### 3 - 1 -Application à l'étude du cytochrome c

### ❖ Evaluation de l'effet de discrimination HCCA/HCCE

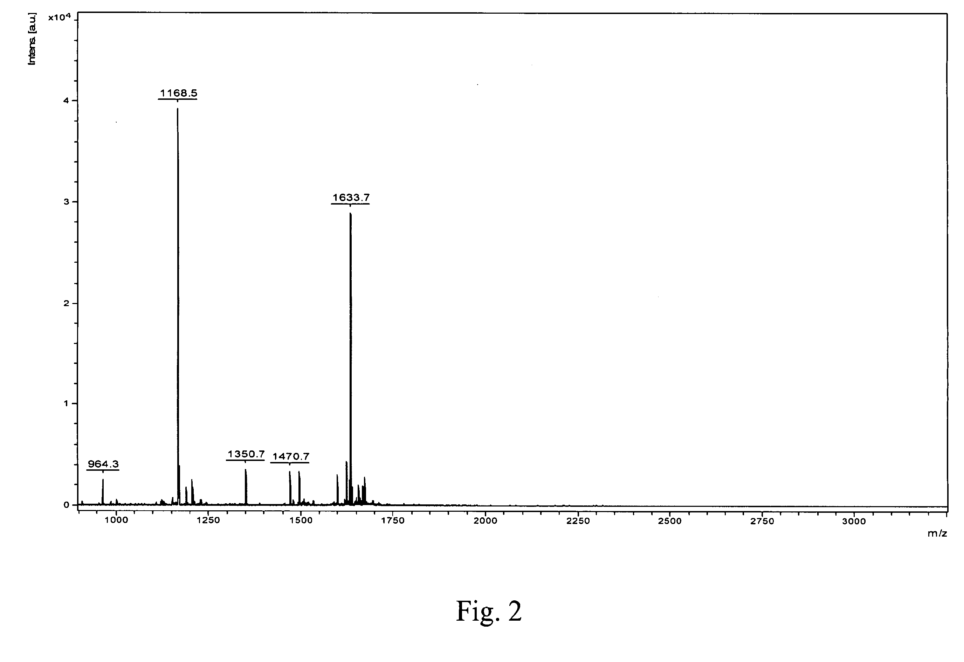

Un spectre de masse MALDI-TOF de référence du cytochrome c non modifié digéré avec de la trypsine et effectuée avec de la matrice HCCA en dépôt goutte séchée, est présenté ci-dessous (cf. Figure **2**).

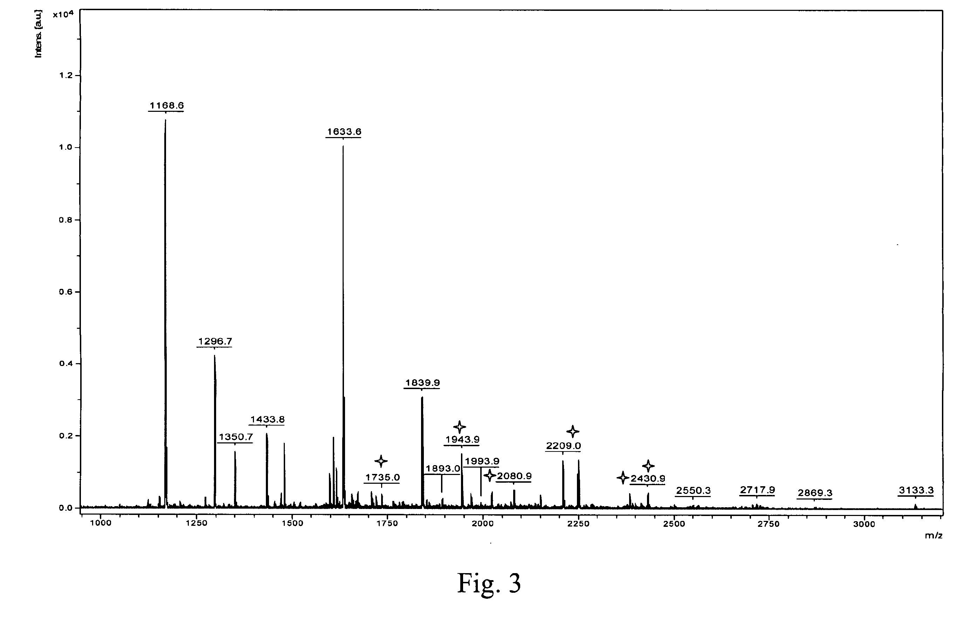

Le spectre de masse du mélange peptidique issu de la digestion trypsique de l'échantillon réticulé précédente ([Cytoc] = 100 µM et R = 0,5) préparé dans la matrice acide HCCA en dépôt goutte séchée est présenté dans la figure **3**.

Six signaux non apparents sur le spectre de référence de la digestion du cytochrome c non modifié sont visibles sur ce spectre avec une intensité relative assez faible. Des signaux correspondant à des peptides non modifiés (*e.g.* m/z : 1296, 1633,1840 Da) sont détectés avec des intensités relatives plus hautes que celles des signaux nouvellement apparus.

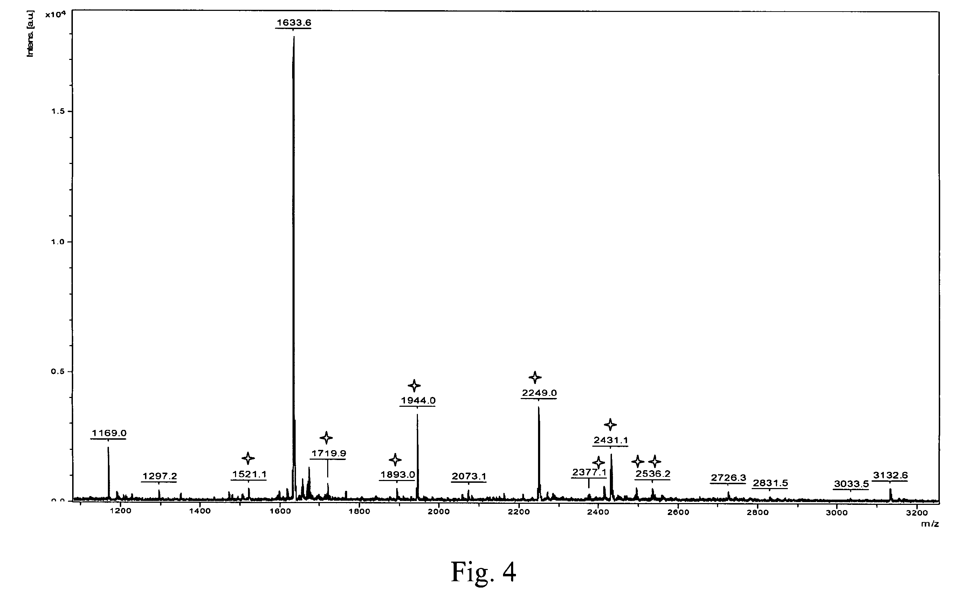

Le même échantillon a été analysé en utilisant la matrice neutre HCCE en dépôt de type couche mince. Le spectre obtenu est présenté dans la figure **4** ci-dessous.

Ce spectre montre cette fois l'apparition de 9 signaux non détectés dans le spectre de référence de la digestion du cytochrome c non modifié et donc trois de plus qu'avec la matrice HCCA. De plus, certains de ions sont détectés avec une meilleure résolution que lorsque la matrice HCCA est utilisée (*e.g.* m/z : 1944 et 2249 Da). Enfin, nombres d'ions correspondant à des peptides non modifiés sont peu visibles lors que la matrice HCCE est utilisée (*e.g.* m/z : 1297, 1351, 1433, 1840 Da). Seul l'ion de valeur m/z 1633 Da correspondant au peptide non modifié préchargé grâce à l'hème complexant un atome de fer est encore détecté avec une bonne résolution.

Cette analyse montre donc que la réticulation effectuée avec le JMV3378 permet d'obtenir un bon effet de discrimination HCCA/HCCE et facilite la détection de peptides modifiés d'intérêt.

La liste de ces nouveaux ions détectés a été soumise au logiciel de prédiction MSX-3D. Les résultats sont présentés dans le tableau suivant.

**Tableau 1 : Liste de masses expérimentales et prédites de peptides réticulés par le JMV3378.**

| [M + H]⁺ | | Déviation | Séquence | Séquence | Modification(s) |
|---|---|---|---|---|---|
| Expérimental | Calculé | (ppm) | 1 | 2 | |
| 1521,06 | 1519,72 | -879 | 6 - 13 | | 2 × T0 |
| 1719,87 | 1719,84 | -20 | 26 - 38 | | T0 |
| 1893,01 | 1892,98 | -18 | 87 - 99 | | T0 |
| | 1892,98 | -18 | 88 - 91 | 92 - 100 | T2 |
| | 1892,98 | -18 | 88 - 100 | | T0 |
| 1943,99 | 1943,96 | -14 | 23 - 38 | | T1 |
| | 2248,03 | -435 | 23 - 38 | | 2 × T0 |
| | 2248,99 | -7 | 9 - 22 | | - |
| 2249,01 | | | | | |
| | 2249,11 | 45 | 73 - 87 | | T0 + T1 |
| | 2249,11 | 45 | 74 - 88 | | T0 + T1 |
| 2413,07 | 2413,04 | -14 | 39 - 55 | | 2 × T0 |
| | 2495,18 | -44 | 56 - 73 | | T0 |
| | 2495,35 | 23 | 73 - 79 | 80 - 91 | T2 |
| 2495,29 | | | | | |
| | 2495,35 | 23 | 73 - 86 | 87 - 91 | T2 |
| | 2495,35 | 23 | 73 - 87 | 88 - 91 | T2 |
| | 2535,05 | -444 | 9 - 22 | | T0 |
| 2536,18 | | | | | |
| | 2536,34 | 64 | 6 - 13 | 80 - 88 | T0 + T2 |

Les signaux détectés peuvent correspondre à différents types de réticulations de peptides (type 0, 1 et 2). Après avoir vérifié les distances théoriques entre les chaînes latérales des lysines du cytochrome c, un total de 8 possibilités de réaction de réticulation de type 1 et 2 ont été répertoriées (cf. schéma 12).

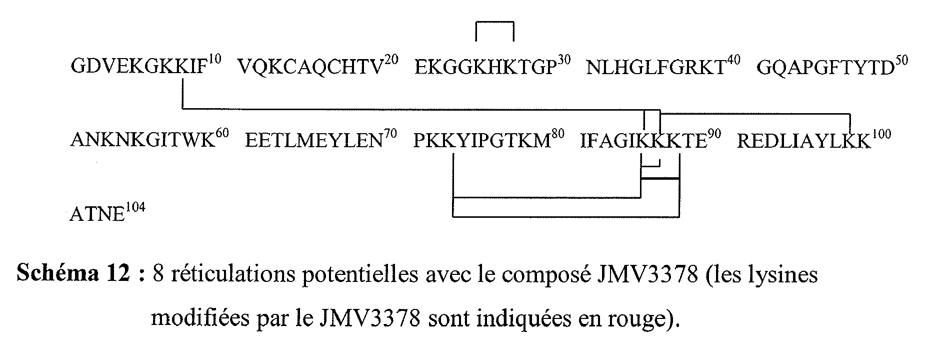

Ces résultats ont mis en évidence que certaines chaînes latérales de lysines (Lys 5, 55, 100) n'ayant pas réagi avec le composé JMV3378 sont peu accessibles.

### 3 - 2 - Application à l 'étude de l'apomyoglobine

Des expériences de réticulation avec le composé JMV3378 ont été réalisées avec l'apomyoglobine. Les tests ainsi que les analyses par spectrométrie de masse MALDI-TOF ont été réalisées selon les mêmes protocoles que pour les expériences effectuées sur le cytochrome c.

### ❖ Evaluation de l'effet de discrimination HCCA/HCCE

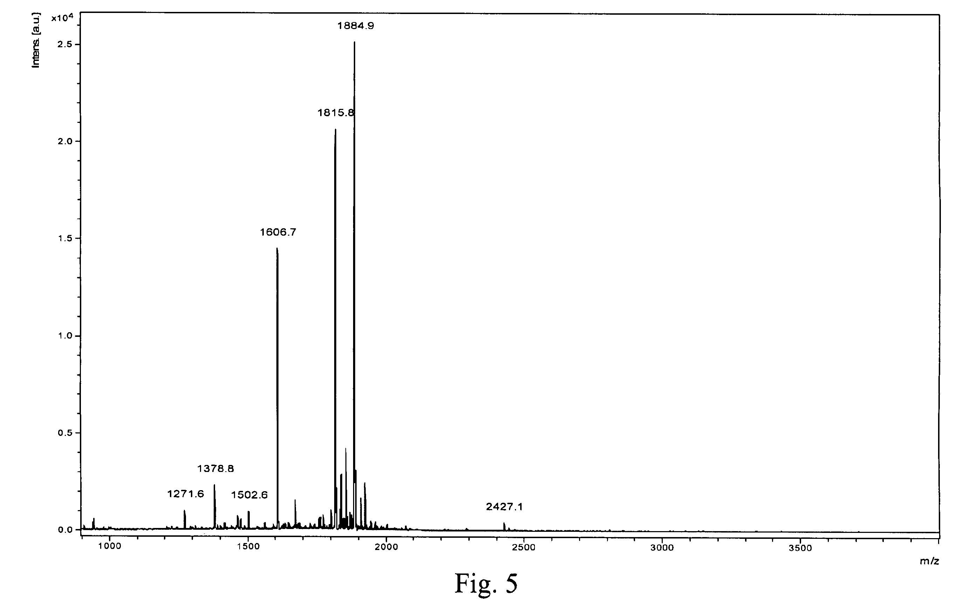

En point de comparaison pour les spectres obtenus avec les digestions enzymatiques des expériences de réticulation, l'analyse d'un mélange peptidique issu d'une digestion trypsique de l'apomyoglobine non modifié est présentée dans la figure **5** ci-dessous.

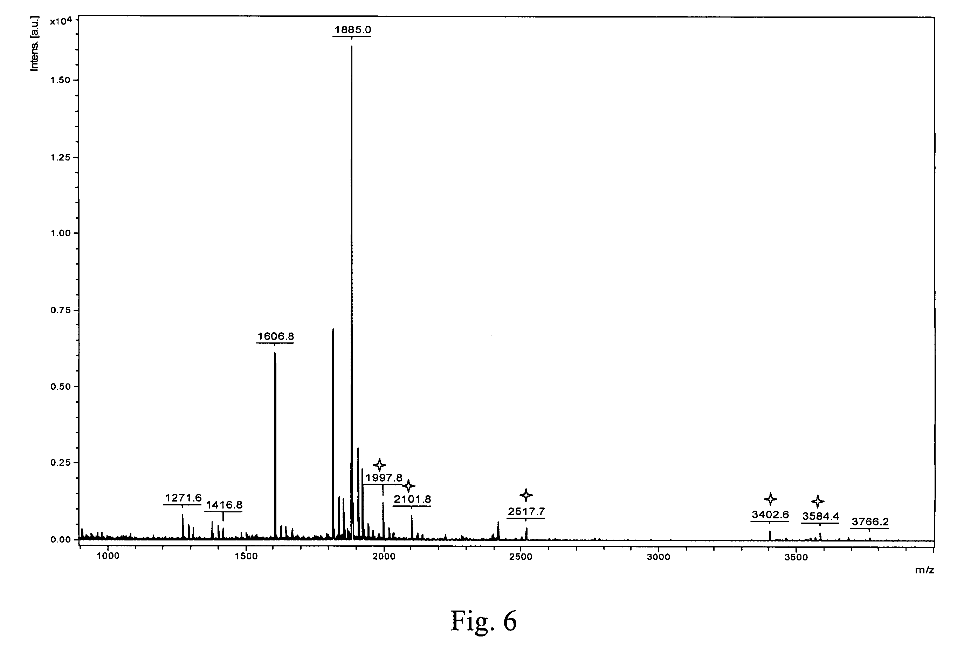

L'expérience de réticulation précédente a été digérée avec de la trypsine puis analysée une première fois avec la matrice acide HCCA (cf. Figure 6).

Une comparaison directe avec l'analyse du mélange peptidique issu de la digestion du l'apomyoglobine native montre que l'on observe 5 nouveaux signaux peu intenses avec la matrice HCCA.

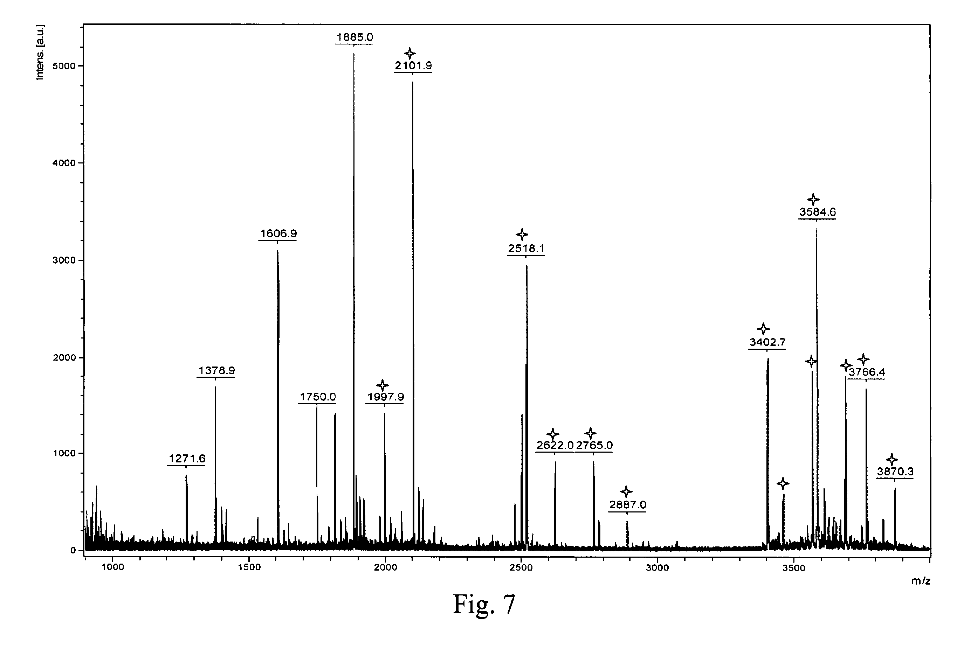

Le même échantillon a été analysé une deuxième fois avec la matrice neutre HCCE (cf. Figure 7).

Dans ce cas, 13 nouveaux signaux intenses peuvent être nettement observés grâce à l'effet de discrimination de détection de peptides réticulés avec le composé JMV3378 et à l'utilisation de la matrice neutre HCCE. De même, l'intensité du signal de valeur m/z 1885 correspondant à un peptide non modifié a diminué de manière conséquente.

Après avoir analysé avec le logiciel MSX-3D les masses correspondant aux nouveaux signaux observés, une liste de prédiction a été obtenue et est présentée dans le tableau **2** ci-dessous.

**Tableau 2 : Liste de masses expérimentales et prédites de peptides réticulés par le JMV3378.**

| [M + H]⁺ | | Déviation | Séquence | Séquence | Modification(s) |
|---|---|---|---|---|---|
| Expérimental | Calculé | (ppm) | 1 | 2 | |
| 1997,89 | 1997,92 | 16 | 48 - 62 | | T1 |
| 2101,89 | 2101,96 | 33 | 1 - 16 | | T0 |
| 2518,08 | 2518,14 | 23 | 119 -139 | | T0 |
| 2622,03 | 2622,39 | 136 | 99 - 118 | | T0 |
| 2765,04 | 2765,32 | 103 | 32 - 47 | 78 - 79 | T0 + T1 |
| 2886,96 | 2887,37 | 141 | 32 - 50 | | T0 |
| 3402,70 | 3402,78 | 23 | 48 - 56 | 63 - 79 | T0 + T2 |
| 3460,63 | 3462,64 | 581 | 57 - 63 | 80 - 98 | T0 + T2 |
| 3566,57 | 3566,74 3566,93 | 46 101 | 46 - 56 57 - 78 | 134 - 147 97 - 102 | T0 + T2 T0 + T2 |
| 3584,56 | 3583,96 3584,77 | -168 57 | 63 - 79 43 - 62 | 140 - 153 140 - 147 | T2 T2 |
| 3688,47 | 3688,84 | 99 | 48 - 56 | 63 - 79 | 2 × T0 + T2 |
| 3766,42 | 3766,08 | -89 | 46 - 50 | 78 - 102 | T2 |
| | 3766,70 | 74 | 46 - 62 | 140 - 147 | 2 × T0 + T2 |
| 3870,29 | 3869,87 | -106 | 78 - 98 | 146 - 153 | T0 + T2 |
| | 3870,02 | -68 | 63 - 79 | 140 - 153 | T0 + T2 |

Les données fournies par cette analyse ont été vérifiées avec la structure cristalline de la protéine. Un total de 7 possibilités de réticulation (type 1 et 2) en accord avec la structure cristalline modèle de la protéine ont été répertoriées (cf. schéma 13).

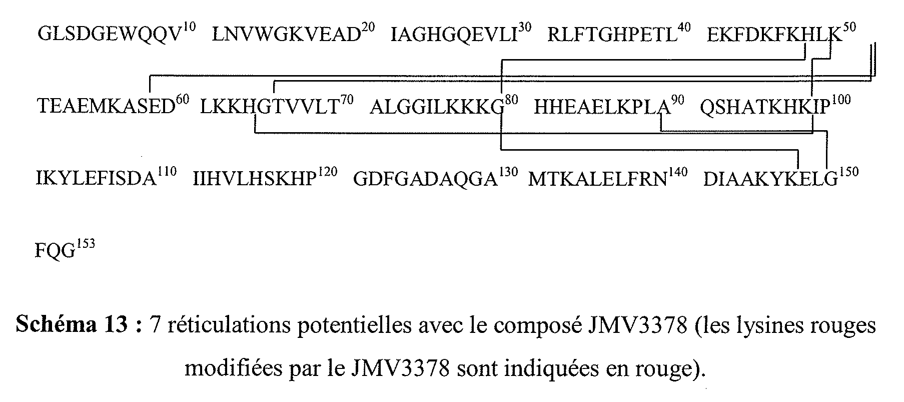

De plus, d'après les prédictions, 16 lysines ont été modifiées avec le composé JMV3378. Les lysines 16 et 118 n'ont pas été données comme accessibles.

L'identification par spectrométrie de masse MALDI-TOF/TOF des peptides réticulés est en cours.

### Exemple 4 : Partie expérimentale

### 4-1 Analyses par spectrométrie de masse MALDI-TOF

Le spectromètre de masse qui a été utilisé est de la gamme Bruker : Ultraflex Daltonics. Les logiciels d'acquisition de spectre et d'exploitation de données sont respectivement : Flexcontrol et Flexanalysis version 2.2.

Le spectromètre est équipé d'une source SCOUT et la désorption/ionisation est effectuée à l'aide d'un laser à azote de longueur d'onde 337 nm et la fréquence utilisée est de 50 Hz. La puissance du laser est modulable grâce à un atténuateur.

### 4 - 1 -1 L'étalonnage

L'étalonnage des analyses par spectrométrie MALDI - TOF a été effectué en mode externe. Deux kits d'étalonnage ont été utilisés :
- « Peptide calibration standard II » (Bruker #222570) : allant de 700 à 3200 Th
- « Protein calibration standard I » (Bruker #206355) : allant de 5000 à 17500 Th

L'étalonnage externe est effectué en faisant un dépôt de l'un de ces mélanges commerciaux avec une matrice appropriée (*e.g.* acide α-cyano-4-hydroxycinnamique (HCCA) pour le « Peptide calibration standard II », acide sinapinique (AS) pour le « Protein calibration standard I ») à proximité du ou des dépôt(s) à analyser.

### 4 -1 - 2 - Préparation de l'échantillon

Les échantillons sont déposés sur une plaque Bruker Daltonics MTP 384 cibles en acier poli, suivant deux méthodes.

### ❖ Le dépôt en goutte séchée

Le dépôt goutte séchée a été préféré dans la plupart des analyses utilisant la matrice HCCA. Il est réalisé par co-cristallisation d'une goutte de solution matricielle et d'une goutte d'une solution d'analyte. Chaque dépôt peut contenir entre 0,5 et 1 µL de chaque solution.

Les solutions de matrice HCCA sont préparées par saturation dans une solution eau/ACN/TFA (50 : 50 : 0,1).
- Analyses avec la matrice acide HCCA de tests de modifications sur protéines après digestion enzymatique :

0,5 µL (0,5 pmol) de solution peptidique à 1 µM dans un mélange eau/ACN/TFA (50 : 50 : 0,1) sont co-cristallisés avec 0,5 µL d'acide HCCA saturé dans un mélange eau/ACN/TFA (50 : 50 : 0,1).

### ❖ Le dépôt sur couche mince

Le dépôt sur couche mince a été utilisé pour chaque dépôt utilisant la matrice HCCE. Il consiste à déposer 0,5 µL d'une solution saturé dans l'acétone de HCCE en bande sur deux cibles marquées sur la plaque de dépôt.

0,5 µL de solution d'analyte sont déposés sur une cible contenant le dépôt séché de matrice. Du fait de la difficulté à reproduire l'aspect du dépôt par cette méthode, il est préférable de renouveler le dépôt une deuxième fois pour chaque analyse.
- Analyses avec la matrice neutre HCCE de tests de modifications sur protéines après digestion enzymatique :
   0,5 µL (0,5 pmol) de solution peptidique à 1 µM dans un mélange eau/ACN/TFA (50 : 50 : 0,1) sont co-cristallisés sur un dépôt de 0,25 µL de matrice neutre HCCE saturé dans de l'acétone.

### 4-2 Synthèse de peptides modèles

Les peptides modèles JMV3346, JMV3347, JMV3348, JMV3349 et JMV3350 ont été synthétisés manuellement sur support solide selon une stratégie Fmoc. Le peptide 3 a été synthétisé sur support solide selon une stratégie Fmoc à l'aide d'un synthétiseur micro-ondes.

Les trois peptides ont été synthétisés avec comme agent de couplage l'HBTU en présence de NMM sur une résine Fmoc-Rink-Amide - aminométhyl-polystyrène (100-200 mesh, 0,7 mmol/g).

### ○ Synthèse du peptide JMV3346 :

*Code 3 lettres :* H-Arg-Lys-Asn-Gly-Pro-Leu-Ile-Gly-Ala-Phe-NH₂
*Caractérisation du composé :*
Aspect : poudre blanche
Formule brute : C₄₉H₈₂N₁₆O₁₁
Masse molaire moyenne = 1071,28 g/mol
Masse molaire moyenne + 3 TFA = 1413,34 g/mol
Masse molaire monoisotopique = 1070,63 g/mol
Temps de rétention (HPLC) = 1,04 min
[M+2H]²⁺ₑₓₚ : m/z : 536,3 Th

### ○ Synthèse du peptide JMV3347 :

*Code 3 lettres :* CCA-Arg-Lys-Asn-Gly-Pro-Leu-Ile-Gly-Ala-Phe-NH₂
*Caractérisation du composé :*
Aspect : poudre blanche
Formule brute : C₅₉H₈₇N₁₇O₁₂
Masse molaire moyenne = 1226,43 g/mol
Masse molaire moyenne + 3 TFA = 1454,47 g/mol
Masse molaire monoisotopique = 1225,67 g/mol
Temps de rétention (HPLC) = 1,26 min
[M+2H]²⁺ₑₓₚ : m/z : 613,9Th

### ○ Synthèse du peptide JMU3348 :

*Code 3 lettres :* HCCA-Arg-Lys-Asn-Gly-Pro-Leu-Ile-Gly-Ala-Phe-NH₂
*Caractérisation du composé :*
Aspect : poudre blanche
Formule brute : C₅₉H₈₇N₁₇O₁₃
Masse molaire moyenne = 1242,43 g/mol
Masse molaire moyenne + 2 TFA = 1470,47 g/mol
Masse molaire monoisotopique = 1241,67 g/mol
Temps de rétention (HPLC) = 1,17 min
[M+2H]²⁺ₑₓₚ : m/z : 621,9 Th

### ○ Synthèse du peptide JMV3349 :

*Code 3 lettres :* MCCA-Arg-Lys-Asn-Gly-Pro-Leu-Ile-Gly-Ala-Phe-NH₂
*Caractérisation du composé :*
Aspect : poudre blanche
Formule brute : C₆₀H₈₉N₁₇O₁₃
Masse molaire moyenne = 1256,45 g/mol
Masse molaire moyenne + 2 TFA = 1484,49 g/mol
Masse molaire monoisotopique = 1255,69 g/mol
Temps de rétention (HPLC) = 1,20 min
[M+2H]²⁺ₑₓₚ : m/z : 629,0 Th

### ○ Synthèse du peptide JMV3350 :

*Code 3 lettres :* ACCA-Arg-Lys-Asn-Gly-Pro-Leu-Ile-Gly-Ala-Phe-NH₂
*Caractérisation du composé :*
Aspect : poudre blanche
Formule brute : C₆₁H₈₉N₁₇O₁₄
Masse molaire moyenne = 1284,46 g/mol
Masse molaire moyenne + 2 TFA = 1512,50 g/mol
Masse molaire monoisotopique = 1283,68 g/mol
Temps de rétention (HPLC) = 1,26 min
[M+2H]²⁺ₑₓₚ : m/z : 643,1 Th

### ○ Synthèse du peptide 3 :

*Code 3 lettres :* H-Arg-Lys-Gly-Lys-Leu-Ala-Phe-NH₂
*Caractérisation du composé :*
Aspect : poudre blanche
Formule brute : C₃₈H₆₇N₁₃O₇
Masse molaire moyenne = 818,02 g/mol
Masse molaire moyenne + 4 TFA = 1274,10 g/mol
Masse molaire monoisotopique = 817,53 g/mol
Temps de rétention (HPLC) = 0,91 min
[M+H]⁺ₑₓₚ : m/z : 818,7 Da

### 4-3 Synthèse des agents bifonctionnels en solution

### 4 - 3 - 1 - Synthèse du N-(α-cyano-4-hydroxycinnamyl)iminodiacétate de N-hydroxysuccinimidyle (JMV3378)

### ○ Synthèse de l'acide N'-(benzyloxycarbony/)iminodiacétique composé 1) :

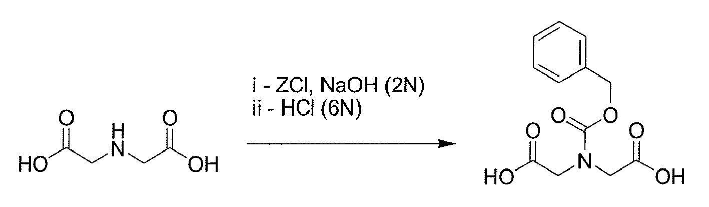

### Tableau récapitulatif des réactifs :

| Réactifs | PM (g/mol) | n (mmol) | éq | m (g) | v (mL) | d (g/mL) |
|---|---|---|---|---|---|---|
| Acide iminodiacétique | 133,1 | 7,50 | 1,0 | 0.999 | - | - |
| ZCl | 170,60 | 28,02 | 3,7 | 4,780 | 4,0 | 1,195 |

### Protocole de synthèse :

0,999 g d'acide iminodiacétique sont dissous sous agitation magnétique dans 7,5 mL d'une solution aqueuse de soude 2N. Le pH du milieu est alors supérieur à 10. Le milieu est placé dans un bain de glace afin de stabiliser sa température à 0°C. 2 fois 2 mL de chloroformiate de benzyle sont successivement ajoutés. Le pH du milieu à 10 est maintenu par l'ajout d'environ 7 mL d'une solution aqueuse de soude 2N.

La réaction est laissée sous agitation pendant 2 jours puis le milieu est extrait avec 4 fois 100 mL d'éther diéthylique. Un flux d'argon est mis en place dans le milieu qui est en suite acidifié avec une solution aqueuse d'HCl 6N jusqu'à atteindre un pH compris entre 1 et 3. Un gel blanc apparaît alors.

Le milieu est alors extrait par 4 fois 100 mL d'AE. Les phases organiques sont rassemblées et séchées avec du MgSO₄ puis filtré sur fritté. Le solvant est évaporé sous pression réduite.

2 g (28 mmol) d'une huile incolore sont récupérés.

### Caractérisation du composé :

Aspect : huile incolore
Rendement = 100%
Formule brute : C₁₂H₁₃NO₆
Masse molaire moyenne = 267,23 g/mol
Masse molaire monoisotopique = 267,07 g/mol
Temps de rétention (HPLC) = 1,18 min
[M+H]⁺ₑₓₚ : m/z : 268,2 Da
Rf (DCM/MeOH/AA - 90 : 10: 0,1; A B) = 0,2

RMN ¹H (300 MHz, CDCl₃, 300°K) : δ(ppm) 4,07 (2H, s, CH₂-CO) ; 4,12 (2H, s, CH₂-CO); 5,11 (2H, s, CH₂-∅); 7,27 (5H, s large, phényle); 7,86 (2H, s large, CO₂H).

RMN ¹³C, Jmod (75 MHz, CDCl₃, 300°K) : δ(ppm) 50,4 (CH₂-CO) ; 68,4 (CH₂-∅) ; 127,8 (C_{2,6} phényle) ; 128,2 (C₄ phényle) ; 128,5 (C_{3,5} phényle) ; 135,5 (C₁ phényle) ; 156,2 (N-CO) ; 173,2 (CO₂H).

### ○ Synthèse du N-(benzyloxvcarbonyl)iminodiacétate de tert-butyle (composé 2) :

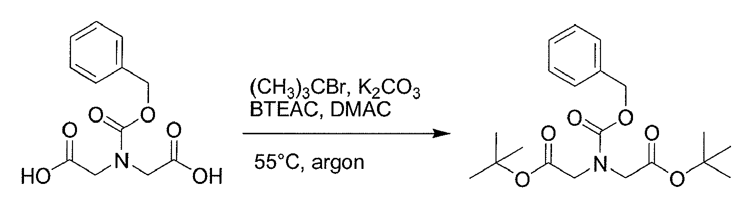

### Tableau récapitulatif des réactifs :

| Réactifs | PM (g/mol) | n (mmol) | éq | m (g) | v (mL) | d (g/mL) |
|---|---|---|---|---|---|---|
| Composé **1** | 267,23 | 2,99 | 1,0 | 0,800 | - | - |
| Bromure de *tert*-butyle | 137,03 | 266,22 | 89,0 | 36,480 | 30,000 | 1,216 |
| K₂CO₃ | 138.00 | 72,46 | 24,2 | 10,000 | - | - |
| BTEAC | 227,78 | 7,20 | 2,4 | 1,640 | - | - |

### Protocole de synthèse :

0,800 g de composé **1** sont dissous en présence de 1,640 g de chlorure de benzyltriéthylammonium dans 75 mL de DMAC sous argon. 10 g de K₂CO₃ sont introduits dans le milieu puis 30 mL de bromure de *tert*-butyle sont ajoutés.

Le milieu est laissé sous agitation magnétique vive à une température de 55°C pendant 30 heures. Le milieu est refroidi à l'air libre jusqu'à température ambiante.

Après 15 jours, le milieu est placé dans un bain de glace puis 400 mL d'eau froide sont ajoutés. La phase aqueuse est extraite avec 5 fois 100 mL d'AE. Chaque phase organique est alors extraite avec 3 fois 50 mL d'une solution aqueuse de NaHCO₃ saturée et 2 fois 50 mL d'eau. Les phases organiques rassemblées sont séchées avec du MgSO₄, filtré sur fritté puis le solvant est évaporé sous pression réduite.

Le composé est purifié par chromatographie sur colonne avec comme éluant : DCM/MeOH/AA (98 : 2 : 0,1).
193 mg (0,51 mmol) d'une huile incolore sont récupérés.

### Caractérisation du composé :

Aspect : huile incolore
Rendement = 17%
Formule brute : C₂₀H₂₉NO₆
Masse molaire moyenne = 379,45 g/mol
Masse molaire monoisotopique = 379,20 g/mol
Temps de rétention (HPLC) = 1,99 min
[M+H]⁺ₑₓₚ : m/z : 380,1 Da
Rf (DCM/MeOH/AA - 90 : 10: 0,1 ; A B) = 0,86

RMN ¹H (300 MHz, CDCl₃, 300°K) : δ(ppm) 1,40 (9H, s, CH₃ *tert*-butyle) ; 1,46 (9H, s, CH₃ *tert*-butyle) ; 3,96 (2H, s, CH₂-CO) ; 4,05 (2H, s, CH₂-CO) ; 5,15 (2H, s, CH₂-∅) ; 7,32 (5H, s large, phényle).

RMN ¹³C, Jmod (75 MHz, CDCl₃, 300°K) : δ(ppm) 27,9 (CH₃ *tert*-butyle) ; 28,1 (CH₃ *tert*-butyle) ; 49,9 (CH₂-CO) ; 50,2 (CH₂-CO) ; 67,7 (CH₂-∅); 81,9 (Cquaternaire *tert*-butyle) ; 127,7 (C_{2,6} phényle) ; 127,9 (C₄ phényle) ; 128,4 (C_{3,5} phényle) ; 136,3 (C₁ phényle) ; 168,5 (N-CO) ; 168,7 (CO₂tBu).

### ○ Synthèse de l'iminodiacétate de tert-butyle (composé 4) à partir du composé 2 :

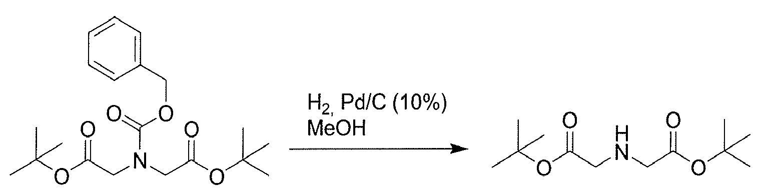

### Tableau récapitulatif des réactifs :

| | | | | | | |
|---|---|---|---|---|---|---|
| Réactifs | PM (g/mol) | n (mmol) | éq | m (g) | v (mL) | d (g/mL) |
| Composé **2** | 379,45 | 0,60 | 1,0 | 0,227 | - | - |

### Protocole de Synthèse :

227 mg de composé **2** sont dissous dans 60 mL de méthanol. 30 mg de palladium sur charbon sont introduits. Un bullage d'hydrogène est mis en place dans le milieu. La réaction est laissée sous agitation magnétique pendant 10 heures.

Le ballon est purgé à l'argon. Le milieu est filtré sur célite et rincé plusieurs fois avec du méthanol.

Le solvant est alors évaporé sous pression réduite puis le milieu est repris avec 100 mL d'AE et extrait avec 2 fois 50 mL d'une solution aqueuse saturée en NaHCO₃ puis 2 fois 50 mL d'eau. La phase organique est séchée avec du MgSO₄ et filtré sur fritté. Le solvant est alors évaporé sous pression réduite.
73 mg (0,3 mmol) d'une huile incolore sont récupérés.

### Caractérisation du composé :

Aspect : huile incolore
Rendement = 50%
Formule brute : C₁₂H₂₃NO₄
Masse molaire moyenne = 245,32 g/mol
Masse molaire monoisotopique = 245,16 g/mol
Temps de rétention (HPLC) = 1,09 min
[M+H]⁺ₑₓₚ : m/z : 246,4 Da
Rf(AcOEt/EP - 2 : 8 ; A B C) = 0,37

RMN ¹H (300 MHz, DMSO-d₆, 300°K) : δ(ppm) 1,41 (9H, s, CH₃ *tert*-butyle) ; 1,42 (9H, s, CH₃ *tert*-butyle) ; 3,32 (4H, s, CH₂-CO).

RMN ¹³C, Jmod (75 MHz, DMSO-d₆, 300°K) : δ(ppm) 27,6 (CH₃ *tert*-butyle) ; 49,5 (CH₂-CO) ; 80,5 (Cquaternaire *tert*-butyle); 170,1 (CO₂tBu).

### ○ Synthèse du N-benzyliminodiacétate de tert-butyle (composé 3) :

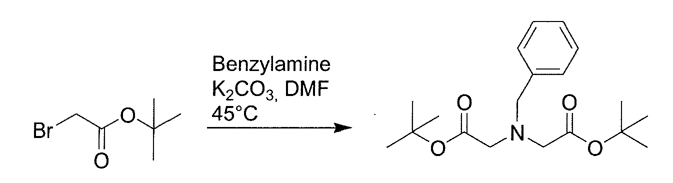

### Tableau récapitulatif des réactifs :

| Réactifs | PM (g/mol) | n (mmol) | éq | m (g) | v (mL) | d (g/mL) |
|---|---|---|---|---|---|---|
| 2-bromoacétate de *tert*-butyle | 195,06 | 35,75 | 3,0 | 6,975 | 5,280 | 1,321 |
| Benzylamine | 107,16 | 11,92 | 1,0 | 1,277 | 1,302 | 0,981 |
| K₂CO₃ | 138,00 | 64,13 | 5,4 | 8,850 | - | - |

### Protocole de synthèse :

5,280 mL de 2-bromoacétate de *tert*-butyle sont dissous dans 100 mL de DMF. 8,850 g de K₂CO₃ sont ajoutés et le milieu est mis sous agitation magnétique forte à une température de 45°C. 1,302 mL de benzylamine sont ajoutés.

La réaction est arrêtée après 3 heures et le solvant est évaporé sous pression réduite. 400 mL d'eau distillée sont ajoutés au milieu qui est extrait avec 4 fois 250 mL d'AE. Chaque phase organique est lavée avec 200 mL d'eau et 200 mL de saumure. Les phases organiques sont rassemblées et séchées avec du MgSO₄ puis filtré sur fritté.

Le composé est purifié par chromatographie sur colonne avec comme éluant : EP/AE (95 : 5).

3,990 g (11,89 mmol) d'une poudre blanche sont récupérés.

### Caractérisation du composé :

Aspect : poudre blanche
Rendement = 99%
Formule brute : C₁₉H₂₉NO₄
Masse molaire moyenne = 335,44 g/mol
Masse molaire monoisotopique = 335,21 g/mol
Temps de rétention (HPLC) = 1,65 min
[M+H]⁺ₑₓₚ : m/z : 336,1 Da
Rf (AE/EP - 5 : 95 ; A B) = 0,33
Point de fusion = 45-50°C

RMN ¹H (300 MHz, CDCl₃, 300°K) : δ(ppm) 1,44 (18H, s, CH₃ *tert*-butyle) ; 3,39 (4H, s, CH₂-CO) ; 3,88 (2H, s, CH₂-∅) ; 7,31 (5H, m, phényle).

RMN ¹³C, Jmod (75 MHz, CDCl₃, 300°K) : δ(ppm) 28,2 (CH₃ *tert*-butyle) ; 55,2 (CH₂-CO); 57,6 (CH₂-∅); 80,9 (Cquaternaire *tert*-butyle); 127,2 (C₄ phényle) ; 128,8 (C_{3,5} phényle) ; 129,1 (C_{2,6} phényle) ; 138,6 (C₁ phényle) ; 170,6 (CO).

### ○ Synthèse du composé 4 à patir du composé 3 :

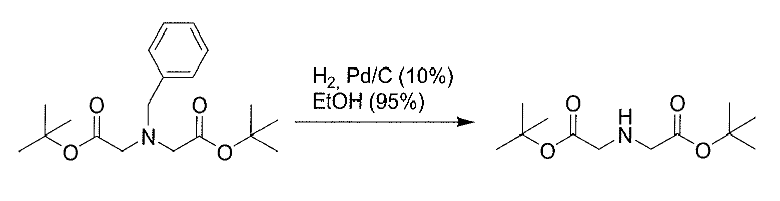

### Tableau récapitulatif des réactifs :

| Réactifs | PM (g/mol) | n (mmol) | éq | m (g) | v (mL) | d (g/mL) |
|---|---|---|---|---|---|---|
| Composé **3** | 335,44 | 23,65 | 1,0 | 7,932 | - | - |

### Protocole de synthèse :

7,932 g de composé **3** sont dissous dans 200 mL d'éthanol à 95% sous agitation magnétique. 200 mg de palladium sur charbon sont introduits dans le milieu. Puis une bullage d'hydrogène est mis en place dans le ballon.

Après **4** heures, le ballon est purgé à l'argon. Le milieu est filtré sur célite et rincé avec du méthanol. Le solvant est alors évaporé sous pression réduite.
5,771 g (23,52 mmol) d'une huile incolore sont récupérés.

### Caractérisation du composé :

Aspect : huile incolore
Rendement = 99,5%
Formule brute : C₁₂H₂₃NO₄
Masse molaire moyenne = 245,32 g/mol
Masse molaire monoisotopique = 245,16 g/mol
Temps de rétention (HPLC) = 1,09 min
[M+H]⁺ₑₓₚ : m/z : 246,4 Da
Rf (AcOEt/EP - 2 : 8 ; A B C) = 0,37

RMN ¹H (300 MHz, DMSO-d₆, 300°K) : δ(ppm) 1,41 (9H, s, CH₃ *tert*-butyle) ; 1,42 (9H, s, CH₃ *tert*-butyle) ; 3,32 (4H, s, CH₂-CO).

RMN ¹³C, Jmod (75 MHz, DMSO-d₆, 300°K) : δ(ppm) 27,6 (CH₃ *tert*-butyle) ; 49,5 (CH₂-CO) ; 80,5 (Cquaternaire *tert*-butyle); 170,1 (CO₂tBu).

### ○ Synthèse du N-(2-chloroacétyl)iminodiacétate de tert-butyle (composé 5) :

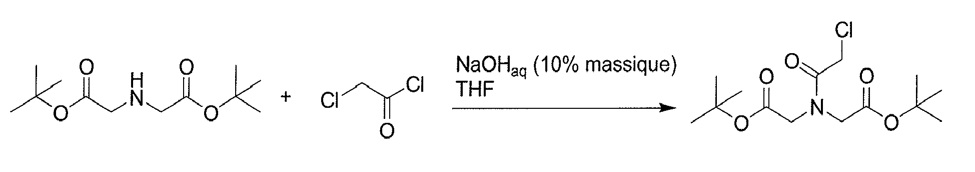

### Tableau récapitulatif des réactifs :

| Réactifs | PM (g/mol) | n (mmol) | éq | m (g) | v (mL) | d (g/mL) |
|---|---|---|---|---|---|---|
| Composé **5** | 245,32 | 5,01 | 1,0 | 1,230 | - | - |
| Chlorure de 2-chloroacétyle | 112,94 | 25,07 | 5,0 | 2,832 | 1,997 | 1,418 |

### Protocole de synthèse :

1,230 g de composé **5** sont dissous dans 40 mL de THF et 3 mL d'une solution aqueuse de soude 2,5 M sont ajoutés. Le milieu est placé sous forte agitation magnétique. 1,997 mL de chlorure de 2-chloroacétyle sont ajoutés lentement et de la soude 1N est régulièrement ajoutée pour maintenir le pH de la solution en dessous de 8.

Après 5 minutes d'agitation, le THF est évaporé sous pression réduite. 150 mL d'AE sont ajoutés au milieu qui est ensuite extrait avec 4 fois 100 mL d'eau distillée. La phase organique est ensuite séchée avec du MgSO₄, filtrée sur fritté et le solvant est évaporé sous pression réduite.
1,605 g (4,99 mmol) d'une huile incolore sont récupérés.

### Caractérisation du composé :

Aspect : huile incolore
Rendement = 99,5%
Formule brute : C₁₄H₂₄ClNO₅
Masse molaire moyenne = 321,80 g/mol
Masse molaire monoisotopique = 321,13 g/mol
Temps de rétention (HPLC) = 1,78 min
[M+H]⁺ₑₓₚ : m/z : 322,4 DA
Rf (AE/EP - 3 : 7 ; A B) = 0,47

RMN ¹H (300 MHz, CDCl₃, 300°K) : δ(ppm) 1,44 (9H, s, CH₃ *tert*-butyle) ; 1,47 (9H, s, CH₃ *tert*-butyle) ; 4,05 (2H, s, CH₂-CO) ; 4,06 (2H, s, CH₂-CO) ; 4,07 (2H, s, CH₂Cl).

RMN ¹³C, Jmod (75 MHz, CDCl₃, 300°K) : δ(ppm) 27,9 (CH₃ *tert*-butyle) ; 27,9 (CH₃ *tert*-butyle) ; 40,7 (CH₂Cl) ; 49,2 (CH₂-CO); 51,7 (CH₂-CO); 82,2 (Cquaternaire *tert-*butyle); 82,9 (Cquaternaire *tert*-butyle) ; 166,9 (N-CO) ; 167,5 (CO₂tBu); 167,7 (CO₂tBu).

### ○ Synthèse du N-(2-cyanoacétyl)iminodiacétate de tert-butyle (composé 6) :

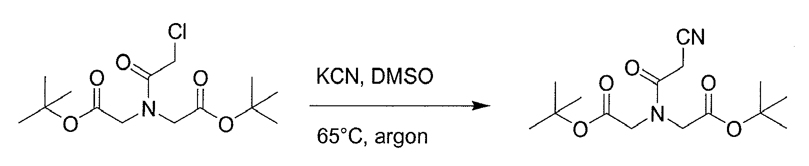

### Tableau récapitulatif des réactifs :

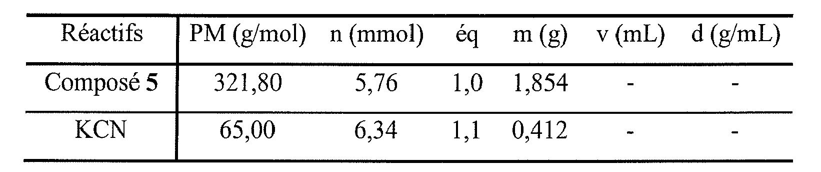

### Protocole de synthèse :

1,854 g de composé **5** sont dissous dans 80 mL de DMSO sous argon et sous agitation magnétique. 412 mg de cyanure de potassium sont ajoutés au milieu qui est mis à une température de 65°C.

La réaction est arrêtée après 1 heure par l'ajout de 400 mL d'eau. Le solvant est ensuite évaporé sous pression réduite. Le milieu est repris avec 200 mL d'eau puis extrait avec 4 fois 200 mL d'AE. Chaque phase organique est lavée avec 250 mL de saumure. Les phases organiques sont rassemblées puis séchées avec du MgSO₄ et filtrées sur fritté. Le solvant est évaporé sous pression réduite.

Le brut réactionnel est purifié par chromatographie sur colonne avec comme éluant un mélange AE/EP (3 : 7).

1,355 g (4,34 mmol) d'une huile incolore sont récupérés.

### Caractérisation du composé :

Aspect : huile incolore
Rendement = 76%
Formule brute : C₁₅H₂₄N₂O₅
Masse molaire moyenne = 312,36 g/mol
Masse molaire monoisotopique = 312,17 g/mol
Temps de rétention (HPLC) = 1,66 min
[M+H]⁺ₑₓₚ : m/z : 313,5 Da
Rf(AE/EP - 3 : 7 ; A B) = 0,37

RMN ¹H (300 MHz, CDCl₃, 300°K) : δ(ppm) 1,45 (9H, s, CH₃ *tert*-butyle) ; 1,48 (9H, s, CH₃ *tert*-butyle) ; 3,55 (2H, s, CH₂-CN) ; 3,97 (2H, s, CH₂-CO) ; 4,06 (2H, s, CH₂-CO).

RMN ¹³C, Jmod (75 MHz, CDCl₃, 300°K) : δ(ppm) 24,7 (CH₂-CN) ; 27,9 (CH₃ *tert-*butyle) ; 27,9 (CH₃ *tert*-butyle) ; 49,5 (CH₂-CO); 51,5 (CH₂-CO); 82,6 (Cquaternaire *tert*-butyle) ; 83,6 (Cquaternaire *tert*-butyle) ; 162,6 (N-CO) ; 166,9 (CO₂tBu); 167,4 (CO₂tBu).

### ○ Synthèse du N-(α-cyano-4-hydroxycinnamyl)iminodiacétate de tert-butyle (Composé 7) :

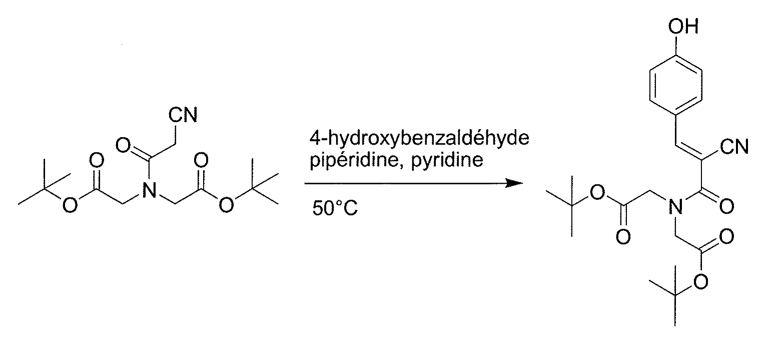

### Tableau récapitulatif des réactifs :

| Réactifs | PM (g/mol) | n (mmol) | éq | m (g) | v (mL) | d (g/mL) |
|---|---|---|---|---|---|---|
| Composé **6** | 312,36 | 0,64 | 1,0 | 0,199 | - | - |
| 4-hydroxy benzaldéhyde | 122,12 | 0,64 | 1,0 | 0,078 | - | - |
| pyridine | 79,10 | 83,20 | 130,0 | 6,581 | 6,729 | 0,978 |
| pipéridine | 85,15 | 0,32 | 0,5 | 0,028 | 0,032 | 0,861 |

### Protocole de synthèse :

199 mg de composé **6** sont dissous dans 6,729 mL de pyridine et 32 µL de pipéridine. 78 mg de 4-hydroxybenzaldéhyde sont ajoutés au milieu sous agitation magnétique à 50°C. Après 15 heures, le solvant est évaporé sous pression réduite. Le milieu est alors repris avec 100 mL d'AE puis extrait avec 4 fois 100 mL d'une solution aqueuse de KHSO₄ 1M et 3 fois 50 mL d'une solution aqueuse saturée en sulfite de sodium et acidifiée avec quelques millilitres d'acide acétique. Un dernier lavage est effectué avec 3 fois 100 mL de saumure. La phase organique est séchée avec du MgSO₄, filtrée sur fritté et le solvant est évaporé sous pression réduite. 258 mg (0,47 mmol) d'une poudre jaune sont récupérés.

### Caractérisation du composé :

Aspect : poudre jaune
Rendement = 74%
Formule brute : C₂₂H₂₈N₂O₆
Masse molaire moyenne = 416,47 g/mol
Masse molaire monoisotopique = 416,19 g/mol
Temps de rétention (HPLC) = 1,85 min
[M+H]⁺ₑₓₚ : m/z : 417,38 Da
Rf(CHCl₃/MeOH/AA - 120 : 10 : 5 ; A B) = 0,43
Point de fusion = 57-59°C

RMN ¹H (300 MHz, CDCl₃, 300°K) : δ(ppm) 1,49 (9H, s, CH₃ *tert*-butyle) ; 1,50 (9H, s, CH₃ *tert*-butyle) ; 4,16 (2H, s, CH₂-CO) ; 4,21 (2H, s, CH₂-CO) ; 6,64 (2H, d, Jₒ = 9Hz, H_{6,8} cinnamyle) ; 7,20 (2H, d, Jₒ = 9Hz, H_{5,9} cinnamyle) ; 7,67 (1H, s, H₃ cinnamyle).

RMN ¹³C, Jmod (75 MHz, CDCl₃, 300°K) : δ(ppm) 27,9 (CH₃ *tert*-butyle) ; 28,1 (CH₃ *tert*-butyle) ; 48,7 (CH₂-CO) ; 51,5 (CH₂-CO) ; 82,8 (Cquaternaire *tert*-butyle) ; 102,2 (C₂ cinnamyle) ; 116,3 (C_{6,8} cinnamyle) ; 116,6 (CN) ; 123,9 (C₄ cinnamyle) ; 132,6 (C_{5,9} cinnamyle) ; 149,8 (C₃ cinnamyle); 159,9 (C₇ cinnamyle) ; 164,5 (C₁ cinnamyle) ; 166,8 (CO₂tBu) ; 167,4 (CO₂tBu).

### ○ Synthèse de l'acide N'-(α-cyano-4-hydroxycinnamyl)iminodiacétique (composé 8) :

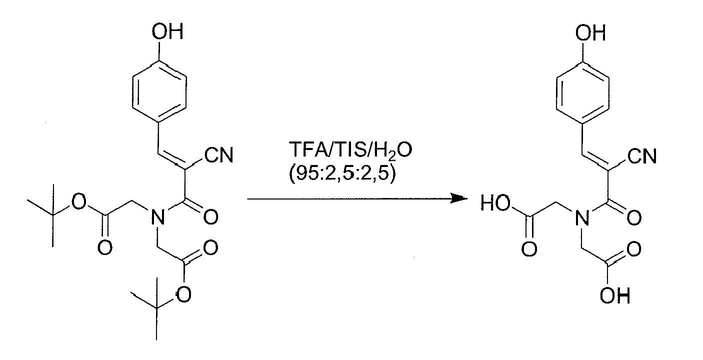

### Tableau récapitulatif des réactifs :

| Réactifs | PM (g/mol) | n (mmol) | éq | m (g) | v (mL) | d (g/mL) |
|---|---|---|---|---|---|---|
| Composé **7** | 416,47 | 0,60 | 1,0 | 0,250 | - | - |

### Protocole de synthèse :

250 mg de composé **7** sont dissous dans 50 mL d'un mélange TFA/TIS/H₂O (95 : 2,5 : 2,5) sous agitation magnétique. Après 1 heure, le solvant est évaporé sous pression réduite. Le milieu est repris dans 50 mL d'un mélange eau/ACN (50 : 50), congelé avec de l'azote liquide et lyophilisé.

150 mg (0,49 mmol) d'une poudre jaune pâle sont récupérés.

### Caractérisation du composé :

Aspect : poudre jaune pâle
Rendement = 82%
Formule brute : C₁₄H₁₂N₂O₆
Masse molaire moyenne = 304,26 g/mol
Masse molaire monoisotopique = 304,07 g/mol
Temps de rétention (HPLC) = 0,89 min
[M+H]⁺ₑₓₚ : m/z : 305,1 Da
Rf(CHCl₃/MeOH/AA - 85 : 10 : 5 ; A B) = 0,47
Point de fusion = 206°C

RMN ¹H (300 MHz, DMSO-d₆, 300°K) : δ(ppm) 4,07 (2H, s, CH₂-CO) ; 4,34 (2H, s, CH₂-CO) ; 6,92 (2H, d, Jₒ = 9Hz, H_{6,8} cinnamyle) ; 7,64 (1H, s, H₃ cinnamyle) ; 7,83 (2H, d, Jₒ = 9Hz, H_{5,9} cinnamyle).

RMN ¹³C, Jmod (75 MHz, DMSO-d₆, 300°K) : δ(ppm) 48,7 (CH₂-CO) ; 51,1 (CH₂-CO) ; 100,0 (C₂ cinnamyle) ; 116,1 (C_{6,8} cinnamyle); 116,4 (CN) ; 123,1 (C₄ cinnamyle) ; 132,5 (C₅,₉ cinnamyle) ; 150,9 (C₃ cinnamyle) ; 161,6 (C₇ cinnamyle) ; 164,8 (C₁ cinnamyle) ; 169,8 (CO₂H).

### ○ Synthèse du N-(α-cyano-4-hydroxycinnamy/)iminodiacétate de N-hydroxysuccinimidyle (JMV3378) :

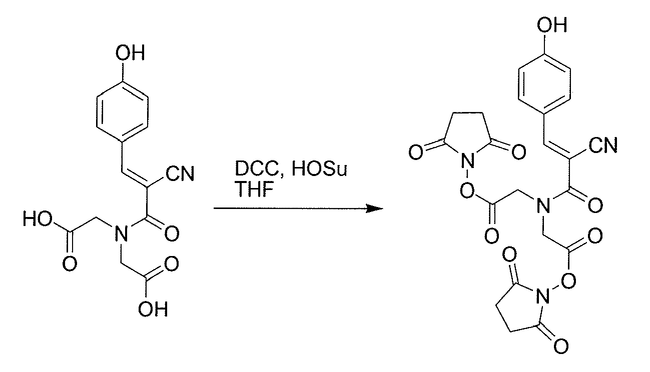

### Tableau récapitulatif des réactifs :

| Réactifs | PM (g/mol) | n (mmol) | éq | m (g) | v (mL) | d (g/mL) |
|---|---|---|---|---|---|---|
| Composé **8** | 304,25 | 2,48 | 1,0 | 0,755 | - | - |
| DCC | 206,30 | 10,25 | 4,1 | 2,115 | - | - |
| HOSu | 115,09 | 6,21 | 2,5 | 0,715 | - | - |

### Protocole de Synthèse :

775 mg de composé **8** sont dissous dans 100 mL de THF et 2,115 g de DCC sont ajoutés. Après 10 minutes, le milieu se trouble et 715 mg d'HOSu sont additionnés. Après 1 heure, le solvant est évaporé sous pression réduite et le milieu est repris avec un minimum de DMF froid et placé dans un bain de glace pendant 10 minutes. La dicyclohexylurée précipite en une poudre blanche. Le milieu est filtré sur un fritté de porosité 4 à froid. Le procédé est répété une deuxième fois. Le solvant est alors évaporé sous pression réduite. Le précipité est dissous dans 300 mL d'AE et extrait avec 2 fois 100 mL d'une solution aqueuse de KHSO₄ 1M. La phase organique est séchée avec du MgSO₄, filtrée sur fritté et le solvant est évaporé sous pression réduite. 0,850 g (1,71 mmol) d'une poudre jaune pâle sont récupérés.

### Caractérisation du composé :

Aspect : poudre jaune pâle
Rendement = 69%
Formule brute : C₂₂H₁₈N₄O₁₀
Masse molaire moyenne = 498.40 g/mol
Masse molaire monoisotopique = 498,10 g/mol
Temps de rétention (HPLC) = 1,27 min
[M+H]⁺ₑₓₚ : m/z : 499,1 Da
Rf(DCM/MeOH - 9 : 1 ; A B) = 0,45

RMN ¹H (300 MHz, DMSO-d₆, 300°K): δ(ppm) 2,82 (8H, s, CH₂ succinimidyle) ; 3,30 (4H, s, N-CH₂-CO); 6,91 (2H, d, Jₒ = 9Hz, H_{6,8} cinnamyle) ; 7,59 (1H, s, H₃ cinnamyle) ; 7,87 (2H, d, Jₒ = 9Hz, H_{5,9} cinnamyle) ; 10,56 (1H, s, OH).

RMN ¹³C, Jmod (75 MHz, DMSO-d₆, 300°K): δ(ppm) 25,7 (CH₂ succinimidyle) ; 25,9 (CH₂ succinimidyle); 60,2 (CH₂-CO); 99,6 (C₂ cinnamyle); 116,5 (C_{6,8} cinnamyle) ; 116,7 (CN) ; 122,8 (C₄ cinnamyle) ; 133,3 (C_{5,9} cinnamyle) ; 151,0 (C₃ cinnamyle) ; 162,4 (C₇ cinnamyle); 165,6 (C₁ cinnamyle); 170.0 (CO succinimidyle) ; 170,2 (CH₂-CO).

### 4 - 3 - 2 - Synthèse du (S)-3-(4-benzoylphényl)-2-(α-cyano-4-hydroxycinnamido)propanoate de N-hydroxysuccinimidyle (JMV3480)

### ○ Synthèse de l'acide (S)-3-(4-benzoylphényl)-2-(α-cyano-4-hydroxycinnamido)propanoique (composé 12) :

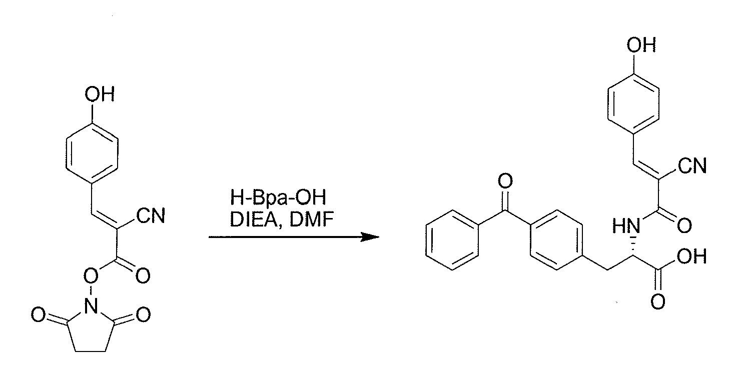

### Tableau récapitulatif des réactifs :

| Réactifs | PM (g/mol) | n (mmol) | éq | m (g) | v (mL) | d (g/mL) |
|---|---|---|---|---|---|---|
| JMV3155 | 286,25 | 0,75 | 1,1 | 0,215 | - | - |
| H-Bpa-OH | 269,30 | 0,68 | 1,0 | 0,183 | - | - |
| DIEA | 129,25 | 0,82 | 1,2 | 0,106 | 0,140 | 0,755 |

### Protocole de synthèse :

183 mg d'acide 4-benzoyl-L-phénylalanine (H-Bpa-OH) sont dissous dans 15 mL de DMF sous agitation magnétique après ajout de 140 µL de DIEA. Après 5 minutes, 215 mg de JMV3155 sont ajoutés au milieu.

Après 24 heures, le solvant est évaporé sous pression réduite. Le milieu est repris avec 200 mL d'AE puis extrait avec 3 fois 80 mL d'une solution aqueuse de KHSO₄ 1M. La phase organique est alors séchée avec du MgSO₄ puis filtrée sur fritté. Le solvant est évaporé sous pression réduite.

Le composé est purifié par HPLC préparative avec comme gradient :
- 0% → 20% ACN en 2 min
- 20% → 50% ACN en 30 min.

Les fractions contenant le composé purifié sont rassemblées, congelées avec de l'azote liquide et lyophilisées.

156 mg (0,36 mmol) d'une poudre jaune sont récupérés.

### Caractérisation du composé :

Aspect : poudre jaune
Rendement = 52%
Formule brute : C₂₆H₂₀N₂O₅
Masse molaire moyenne = 440.45 g/mol
Masse molaire monoisotopique = 440,14 g/mol
Temps de rétention (HPLC) = 1,53 min
[M+H]⁺ₑₓₚ : m/z : 441,0 Da
Rf(CHCl₃/MeOH/AA - 85 : 10 : 5 ; A B) = 0,47

RMN ¹H (300 MHz, DMSO-d₆, 300°K) : δ(ppm) 3,28 - 3,36 (2H, m, H_{β}) ; 4,61 - 4,68 (1H, m, H_{α}) ; 6,67 (2H, d, Jₒ = 9 Hz, H_{6,8} cinnamyle) ; 6,96 (2H, d, Jₒ = 9 Hz, H_{5,9} cinnamyle) ; 7,47 (2H, d, Jₒ = 9 Hz, H_{3,5} *p*-méthylbenzoyle) ; 7,63 - 7,70 (5H, m, H_{2,6} *p*-méthylbenzoyle et H_{3,4,5} benzoyle) ; 7,86 (2H, d, Jₒ = 9 Hz, H_{2,6} benzoyle) ; 7,87 (1H, s, H₃ benzoyle) ; 8,51 (1H, s, OH).

RMN ¹³C, Jmod (75 MHz, DMSO-d₆, 300°K) : δ(ppm) 36,1 (Cp) ; 53,9 (C_{α}) ; 100,7 (C₂ cinnamyle) ; 116,2 (C_{6,8} cinnamyle) ; 116,9 (CN) ; 122,7 (C₄ cinnamyle) ; 128,5 (C_{5,9} cinnamyle) ; 129,3 (C_{3,5} *p*-méthylbenzoyle) ; 129,4 (C_{3,5} benzoyle) ; 129,6 (C p-méthylbenzoyle) ; 132,5 (C₄ benzoyle) ; 132,9 (C_{2,6} benzoyle) ; 135,2 (C₁ *p-*méthylbenzoyle) ; 137,2 (C₁ benzoyle) ; 143,1 (C₄ *p*-méthylbenzoyle) ; 150,7 (C₃ cinnamyle); 161,7 (C₇ cinnamyle) ; 161,9 (C₁ cinnamyle); 172,2 (CO₂H) ; 195,4 (CO-Ø).

### ○ Synthèse du JMV3480 :

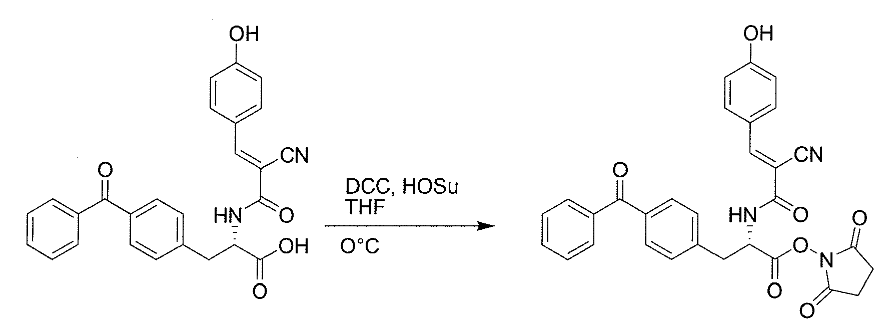

### Tableau récapitulatif des réactifs :

| Réactifs | PM (g/mol) | n (mmol) | éq | m (g) | v (mL) | d (g/mL) |
|---|---|---|---|---|---|---|
| Composé **12** | 440,45 | 0,12 | 1,0 | 0,052 | - | - |
| DCC | 206,30 | 0,28 | 2,3 | 0,058 | - | - |
| HOSu | 115,09 | 0,29 | 2,4 | 0,033 | - | - |

### Protocole de synthèse :

52 mg de composé **12** sont dissous dans 5 mL de THF et 58 mg de DCC sont ajoutés sous agitation magnétique. La température du milieu est baissée à 0°C puis 33 mg de HOSu sont additionnés.

Après 5 heures, la dicyclohexylurée précipité en une poudre blanche est filtrée sur un fritté de porosité 4 à froid. Le solvant est alors évaporé sous pression réduite puis milieu est repris avec 150 mL d'AE et extrait avec 4 fois 100 mL d'une solution aqueuse de KHSO₄ 1M. La phase organique est séchée avec du MgSO₄, filtré sur fritté et le solvant est évaporé sous pression réduite.

60 mg (0,11 mmol) d'une poudre jaune sont récupérés.

### Caractérisation du composé :

Aspect : poudre jaune
Rendement = 93%
Formule brute : C₃₀H₂₃N₃O₇
Masse molaire moyenne = 537,52 g/mol
Masse molaire monoisotopique = 537,15 g/mol
Temps de rétention (HPLC) = 1,69 min
[M⁺H]⁺ₑₓₚ : m/z : 538,2 Da
Rf (CHCl₃/MeOH/AA - 85 : 10 : 5 ; A B) = 0,7

### 4 - 3 - 3 Synthèse de l'acide 5-(α-cyano-4-hydroxycinnamido)isophthalique (composé 11)

### ○ Synthèse de l'acide 5-(2-chloroacétamido)isophthalique (composé 9) :

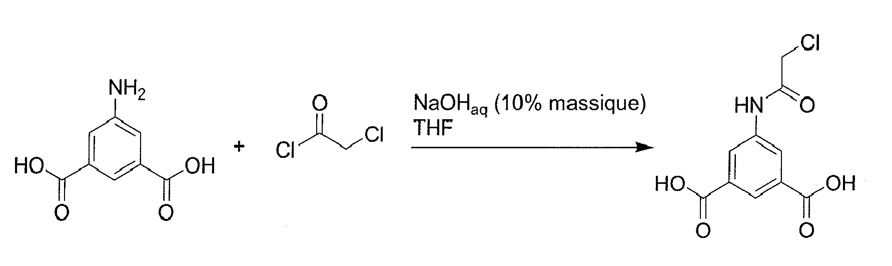

### Tableau récapitulatif des réactifs :

| Réactifs | PM (g/mol) | n (mmol) | éq | m (g) | v (mL) | d (g/mL) |
|---|---|---|---|---|---|---|
| acide 5-amino-isophtalique | 181,15 | 19,41 | 1,0 | 3,516 | - | - |
| chlorure de 2-chloroacétyle | 112,94 | 97,04 | 5,0 | 10,960 | 7,729 | 1,418 |

### Protocole de synthèse :

3,516 d'acide 5-aminoisophtalique sont dissous dans 150 mL de THF contenant 20 mL d'une solution aqueuse de soude à 10% massique. Après 5 minutes sous agitation magnétique, le diacide est totalement solubilisé. 7,729 mL de chlorure de 2-chloroacétyle sont ajoutés lentement dans le milieu et le pH de la solution est maintenu à 10 par ajout d'une solution de soude 1N sous agitation magnétique forte. La réaction est exothermique. Après 5 minutes d'agitation, le THF est évaporé sous pression réduite.

Le milieu est repris avec 400 mL d'AE puis extrait avec 2 fois 100 mL d'une solution aqueuse de KHSO₄ 1M. Les phases aqueuses sont extraites avec 2 fois 100 mL d'AE. Les phases organiques sont rassemblées puis séchées avec du MgSO₄ et filtré sur fritté. Le milieu est concentré jusqu'à saturation de la solution (environ 300 mL). Le composé est alors précipité par l'ajout de 300 mL d'EP. Le solide est filtré sur un fritté de porosité 4.

4,762 g (18,49 mmol) d'une poudre blanche sont récupérés.

### Caractérisation du composé :

Aspect : poudre blanche
Rendement = 96%
Formule brute : C₁₀H₈ClNO₅
Masse molaire moyenne = 257,63 g/mol
Masse molaire monoisotopique = 257,01 g/mol
Temps de rétention (HPLC) = 0,95 min
[M+H]⁺ₑₓₚ : m/z : 258,0 Da
Rf(CHCl₃/MeOH/AA - 40 : 10 : 5 ; A B) = 0,63

RMN ¹H (300 MHz, DMSO-d₆, 300°K) : δ(ppm) 4,29 (2H, s, CH₂Cl) ; 8,20 (1H, s, H₄ 5-aminoisophtalique) ; 8,43 (2H, s, H_{2,6} 5-aminoisophtalique) ; 10,66 (2H, s, CO₂H).

RMN ¹³C, Jmod (75 MHz, DMSO-d₆, 300°K) : δ(ppm) 43,5 (CH₂Cl) ; 123,7 (C_{2,6} 5-aminoisophtalique) ; 125,1 (C₄ 5-aminoisophtalique) ; 131,9 (C_{3,5} 5-aminoisophtalique); 139,1 (C₁ 5-aminoisophtalique); 165,2 (NH-CO); 166,3 (CO₂H).

### ○ Synthèse de l'acide 5-(2-cyanoacétamido)isophthalique (composé 10) :

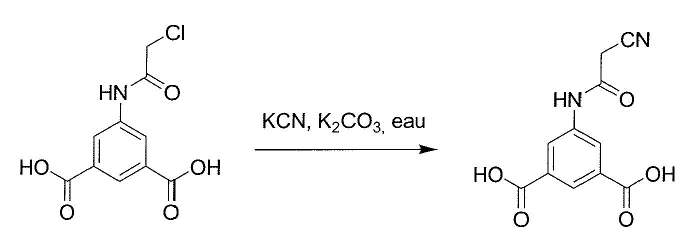

### Tableau récapitulatif des réactifs :

| Réactifs | PM (g/mol) | n (mmol) | éq | m (g) | v (mL) | d (g/mL) |
|---|---|---|---|---|---|---|
| Composé **9** | 257,63 | 18,48 | 1,0 | 4,762 | - | - |
| KCN | 65,00 | 74,34 | 4,0 | 4,832 | - | - |
| K₂CO₃ | 138,00 | 27,51 | 1,5 | 3,796 | - | - |

### Protocole de synthèse :

4,762 g de composé **9** sont placés dans 150 mL d'eau distillée et 3,796 g de K₂CO₃ sont ajoutés sous agitation magnétique vive. La solution devient homogène après 5 minutes d'agitation. 4,832 g de cyanure de potassium sont ajoutés lentement. La solution se colore rapidement en rouge.

Après 2 jours, la réaction est arrêtée et le milieu est acidifié par ajout de quelques millilitres d'une solution aqueuse de HCl 6N jusqu'à un pH inférieur à 3. Le milieu est alors extrait avec 8 fois 100 mL d'AE. Chaque phase organique est lavée avec 100 mL d'une solution aqueuse de KHSO₄ 1M. Les phases organiques sont rassemblées et séchées avec du MgSO₄ et filtrées sur fritté. Le solvant est évaporé sous pression réduite.

3,511 g (14,15 mmol) d'une poudre blanche sont récupérés.

### Caractérisation du composé :

Aspect : poudre blanche
Rendement = 77%
Formule brute : C₁₁H₈N₂O₅
Masse molaire moyenne = 248,19 g/mol
Masse molaire monoisotopique = 248,04 g/mol
Temps de rétention (HPLC) = 0,83 min
[M+H]⁺ₑₓₚ : m/z : 249,2 Da
Rf(CHCl₃/MeOH/AA - 40 : 10 : 5 ; A B) = 0,30

RMN ¹H (300 MHz, DMSO-d₆, 300°K) : δ(ppm) 3,94 (2H, s, CH₂-CN) ; 8,20 (1H, s, H₄ 5-aminoisophtalique) ; 8,38 (2H, s, H_{2,6} 5-aminoisophtalique) ; 10,67 (2H, s, CO₂H).

RMN ¹³C, Jmod (75 MHz, DMSO-d₆, 300°K) : δ(ppm) 26,8 (CH₂Cl) ; 115,6 (CN) ; 123,6 (C_{2,6} 5-aminoisophtalique) ; 152,1 (C₄ 5-aminoisophtalique) ; 131,9 (C_{3,5} 5-aminoisophtalique); 138,9 (C₁ 5-aminoisophtalique); 161,6 (NH-CO); 166,3 (CO₂H).

### ○ Synthèse de l'acide 5-(α-cyano-4-hydroxycinnamido)isophthalique (composé 11) :

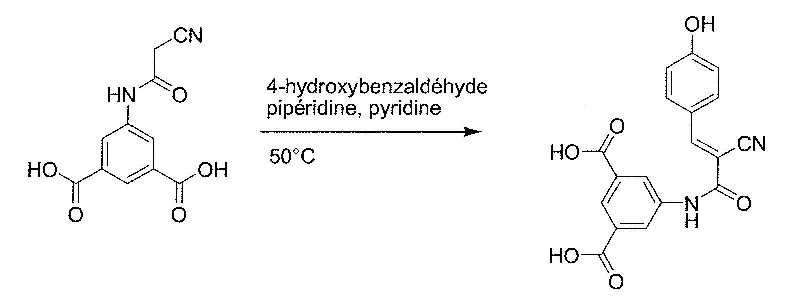

### Tableau récapitulatif des réactifs :

| Réactifs | PM (g/mol) | n (mmol) | éq | m (g) | v (mL) | d (g/mL) |
|---|---|---|---|---|---|---|
| Composé **10** | 248,19 | 2,01 | 1,0 | 0,500 | - | - |
| 4-hydroxy benzaldéhyde | 122,12 | 2,01 | 1,0 | 0,246 | - | - |
| pipéridine | 85,15 | 1,01 | 0,5 | 0,086 | 0,100 | 0,861 |
| pyridine | 79,10 | 262,10 | 130,4 | 20,732 | 21,198 | 0,978 |

### Protocole de synthèse :

500 mg de composé **10** sont dissous dans 200 mL de pyridine et 100 µL de pipéridine sont ajoutés. Après 5 minutes, 246 mg de 4-hydroxybenzaldéhyde sont ajoutés et le milieu est placé dans un bain d'huile à 50°C pendant 3 heures.

Le milieu est refroidi lentement jusqu'à température ambiante. Il est additionné goutte à goutte à 150 mL d'une solution aqueuse de KHSO₄ 1M. Le pH est inférieur à 4. Un précipité jaune apparaît et la solution est triturée avec une spatule. Le précipité est filtré sur un fritté de porosité 4 puis dissous dans du DMF. Le solvant est évaporé sous pression réduite. Le solide est repris dans 150 mL d'une solution aqueuse de KHSO₄ 1M puis trituré et filtré comme précédemment. Le solide est alors dissous dans un mélange eau/ACN 50%, congelé dans l'azote liquide puis lyophilisé.

700 mg (1,99 mmol) d'une poudre jaune sont récupérés.

### Caractérisation du composé :

Aspect : poudre jaune
Rendement = 99%
Formule brute : C₁₈H₁₂N₂O₆
Masse molaire moyenne = 352,30 g/mol
Masse molaire monoisotopique = 352,07 g/mol
Temps de rétention (HPLC) = 1,30 min
[M+H]⁺ₑₓₚ : m/z : 353,0 Da
Rf(CHCl₃/MeOH/AA - 40 : 10 : 5 ; A B) = 0,6

RMN ¹H (300 MHz, DMSO-d₆, 300°K) : δ(ppm) 6,98 (2H, d, Jₒ = 9 Hz, H_{6,8} cinnamyle) ; 7,94 (2H, d, Jₒ = 9 Hz, H_{5,9} cinnamyle) ; 8,23 (2H, s, H_{2,6} 5-aminoisophtalique); 8,55 (1H, s, H₄ 5-aminoisophtalique) ; 8,55 (1H, s, H₃ cinnamyle) ; 10,60 (2H, s, CO₂H).

RMN ¹³C, Jmod (75 MHz, DMSO-d₆, 300°K) : δ(ppm) 101,8 (C₂ cinnamyle) ; 116,4 (C_{6,8} cinnamyle) ; 116,8 (CN); 122,8 (C₄ cinnamyle); 124,9 (C_{2,6} 5-aminoisophtalique) ; 125,3 (C₄ 5-aminoisophtalique) ; 131,7 (C_{3,5} 5-aminoisophtalique) ; 133,1 (C_{5,9} cinnamyle) ; 139,2 (C₁ 5-aminoisophtalique) ; 151,1 (C₃ cinnamyle) ; 161,6 (C₇ cinnamyle) ; 162,1 (C₁ cinnamyle) ; 166,4 (CO₂H).

## Revendications

1. Agent de réticulation des protéines de formule (I) 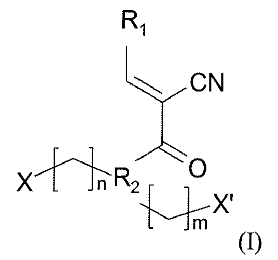 dans laquelle
R₁ est un groupe aryle éventuellement substitué une ou plusieurs fois par un groupement choisi dans le groupe constitué des groupements hydroxy, C₁-C₄ alkyle, OBoc, SO₃Na, Deu, C₁-C₄ alcoxy,
R₂ est N, 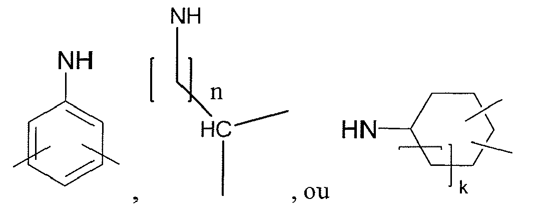
n et m sont des entiers identiques ou différents compris antre 0 et 10, de préférence compris entre 1 et 5
p est un entier compris entre 0 et5,
k est 0, 1, 2 ou 3,
X et X', identiques ou différents, sont une fonction réactive des protéines.

2. Agent de réticulation selon la revendication 1, où n = m.

3. Agent de réticulation selon la revendication 1 ou 2, où R₁ est un groupe phénoxy.

4. Agent de réticulation selon l'une quelconque des revendications précédentes,
où R₂ est

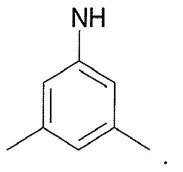

5. Agent de réticulation selon l'une quelconque des revendications précédentes, où X et X', identiques ou différents, sont choisis dans le groupe constitué des fonctions imidoester, ester N-hydroxysuccinimide, isocyanate, isothiocyanate, N-maléimide, disulfure, 1,2-dicarbonyle, benzophénone et arylazide.

6. Agent de réticulation selon l'une quelconque des revendications précédentes, de formule

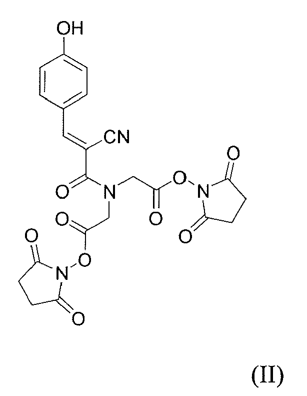

7. Agent de réticulation selon l'une quelconque des revendications précédentes, de formule

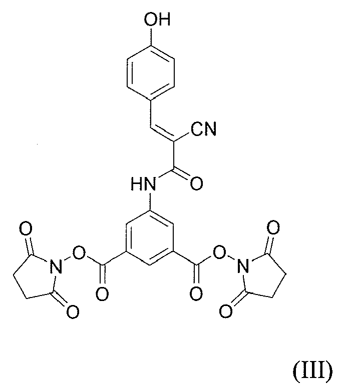

8. Agent de réticulation selon l'une quelconque des revendications précédentes, de formule

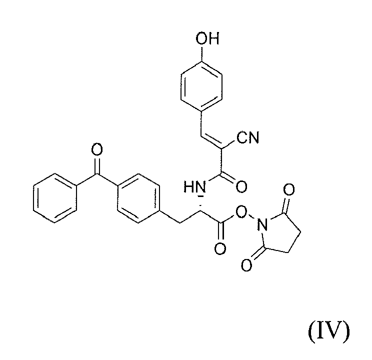

9. Procédé de préparation d'un agent de réticulation selon l'une quelconque des revendications 1 à 8, comprenant les étapes suivantes :
(a) couplage peptidique entre l'amine RR'NH et l'acide carboxylique 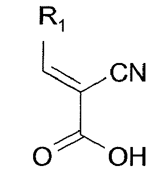 dont la fonction acide est éventuellement activée
pour donner 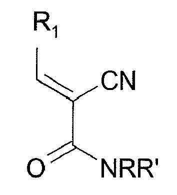 avec R¹ ayant la signification donnée précédemment
avec R et R' représentant :
- tous deux -(alkyl)-COOY
- l'un des deux H et l'autre 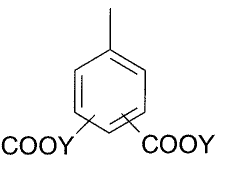
- l'un des deux H et l'autre 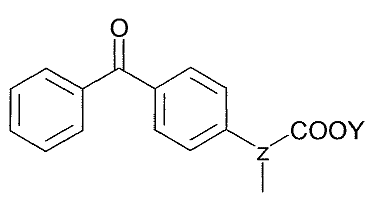 avec Y est H ou tBu,
et Z signifie un groupe alkyle en C₁ à C₈
ou (a') (i) réaction de RR'NH avec 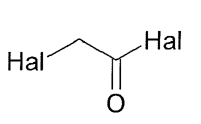 où Hal est un atome d'halogène
en présence d'une base pour donner 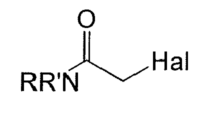
(ii) réaction de 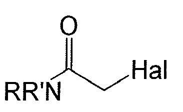 avec un cyanure, tel que KCN, pour donner 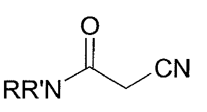
(iii) réaction de 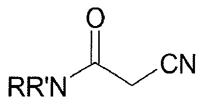 avec R₁CHO en présence d'une base pour donner 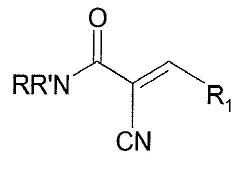
(b) hydrolyse éventuelle de l'ester obtenu à l'étape (a) ou (a')
en acide pour donner 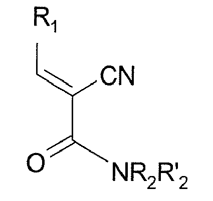 avec R₂ et R₂' représentant :
- tous deux -(alkyl)-COOH
- l'un des deux H et l'autre 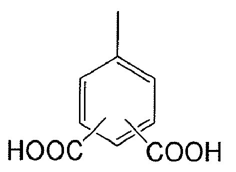
- l'un des deux H et l'autre 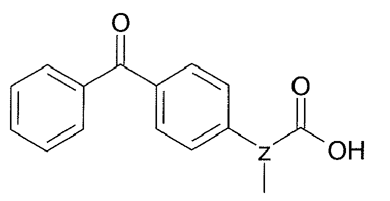
où Z signifie un groupe alkyle en C₁ à C₈
(c) réaction du composé obtenu à l'étape (b) précédente avec une fonction réactive des protéines.

10. Utilisation d'un agent de réticulation selon l'une quelconque des revendications 1 à 8 pour l'analyse de la structure tridimensionnelle d'une protéine en spectrométrie de masse.

11. Méthode d'analyse structurale d'une protéine ou d'un complexe de protéines comprenant les étapes suivantes :
d) réticulation de la protéine ou du complexe protéique sur l'agent de réticulation selon l'une quelconque des revendications 1 à 8 par les fonctions X et/ou X',
e) digestion enzymatique de la protéine ou complexe de protéines fixé à l'agent de réticulation sur selon l'une quelconque des revendications 1 à 8,
f) analyse par spectrométrie de masse.

12. Méthode selon la revendication 11, l'analyse par spectrométrie de masse étant réalisée avec la matrice HCCE.

13. Méthode selon l'une quelconque des revendications 11 à 12, la digestion enzymatique étant réalisée par la trypsine.
